# EUROPEAN PATENT SPECIFICATION

(11) **EP 0 383 129 B1**
(45) Date of publication and mention of the grant of the patent: **10.05.1995**
(21) Application number: 90102179.0
(22) Date of filing: 03.02.1990
(51) Int. Cl.: C12N 15/12, G01N 33/68, A61K 38/17, A61K 35/39, C12P 21/02, C07K 14/435, C07K 16/18

(54) **Pancreatic islet cell antigens obtained by molecular cloning**
Pankreatische Isletzellen-Antigene, erhalten durch molekulare Klonierung
Antigènes de cellules islet pancréatiques obtenus par clonage moléculaire

(30) Priority: 17.02.1989 US 312543; 04.12.1989 US 441703
(43) Date of publication of application: 22.08.1990
(73) Proprietor: Bayer Corporation, Pittsburgh, PA 15219-2502 (US)
(72) Inventor: Rabin, Daniel, Branford, CT 06405 (US)
(74) Representative: Dänner, Klaus, Dr.

(56) References cited:
- EP-A- 0 084 796
- EP-A- 0 286 447
- WO-A-86/07464
- WO-A-88/10120
- GB-A- 2 146 338
- JOURNAL OF CLINICAL INVESTIGATION vol. 79, no. 3, March 1987,pages 926-934, New York, US; S. BAEKKESKOV et al.: "Antibodies to a 64,000 Mr human islet cell antigen precede the clinical onset of insulin-dependentdiabetes"
- idem
- DIABETES vol. 34, no. 6, June 1985, pages 617-619, New York, US; R.C. NAYAK et al.: "'Cytoplasmic' islet cell antibodies. Evidence that the target antigen is a sialoglycoconjugate"
- NATURE vol. 298, 8 July 1982, pages 167-169, London, GB; B. BAEKKESKOV et al.: "Autoantibodies in newly diagnosed diabetic children immunoprecipitate human pancreatic islet cell proteins"
- BIOSIS ACCESSION NO. 82043197 I. MATSUBA et al.: "Studies on the pathogenesis of insulin-dependent diabetes mellitus autoantigensrecognized by islet cell surface antibodies" & J. Jpn. Diabetes Soc. 1986, vol. 29, no. 3, pages 261-265
- DIABETES METABOLISM REVIEWS vol. 3/4, 1987, pages 959-980, US; A. LERNMARK et al.: "Islet-specific immune mechanisms"
- BIOSIS ACCESSION NO. 64050513 W.J. IRVINE et al.:"Pancreatic islet cell antibody as a marker for asymtomatic and latent diabetesand pre diabetes" & Lancet 1976, vol. 2, no.7995, pages 1098-1102
- COMPTES RENDUS DE L'ACADEMIE DES SCIENCES - SCIENCES DE LA VIE - SERIE IIIvol. 309, no. 7, 1989, pages 229-234, Paris, F; C. BOITARDet al.: "Réaction croisée entre les antigènes de classe II du complexe majeurd'histocompatibilité de la souris NOD et un antigène insulaire de poidsmoléculaire de 58kDa"
- SCIENTIFIC AMERICAN July 1990, pages 42-49, New York, US; M.A. ATKINSON et al. "What causes diabetes?"

## Description

### BACKGROUND OF THE INVENTION

This invention relates to pancreatic islet cell antigens that bind with antibodies found in the sera of patients afflicted with insulin-dependent (Type I) diabetes mellitus (IDDM). More particularly, the invention relates to proteins and peptides that bind with islet cell antibodies (ICA) and that are prepared by recombinant DNA (rDNA) or synthetic methods. The invention also concerns cloned DNA encoding such ICA proteins and peptides. The ICA proteins and peptides of the present invention are useful as immunoassay reagents in the presymptomatic diagnosis of IDDM.

The accumulating evidence of cellular and humoral abnormalities associated with IDDM has led to the hypothesis that the disease is an autoimmune disorder. Serum antibodies directed against the insulin-producing beta cells of the pancreatic islets have been detected by immunofluorescence, [G.F. Bottazzo, A. Florin-Christensen, and D. Doniach: Islet Cell Antibodies in Diabetes Mellitus With Autoimmune Polyendocrine Deficiencies, Lancet ii:1279-1283 (1974), and A.C. MacCuish, J. Jordan, C.J. Campbell, L.J.P. Duncan, and W.J. Irvine: Antibodies to Islet-cell in Insulin-dependent Diabetics With Coexistent Autoimmune Disease, Lancet ii:1529-1533 (1974)]. These autoantibodies are observed in 70-80% of newly diagnosed diabetics (NDD), but only in 0.1-1% of normal control subjects [C.H. Brogren and A. Lernmark: Islet Cell Antibodies in Diabetes. Clin. Endocrinol. Metab. 11:409-430 (1982)], and G.F. Bottazzo, R. Pujol-Borrell, and D. Doniach: Humoral and Cellular Immunity in Diabetes Mellitus. Clin. Immunol. Allergy 1:139-159 (1981)]. ICAs have come to be accepted as one predictive factor for IDDM. A review of current knowledge on ICA is provided by A. Lernmark, Diabetic Medicine 4:285-292 (1987).

The conventional ICA assay consists of exposing pancreas sections to sera, staining with a second antibody bearing either a fluorescent [G.F. Bottazzo et al., supra] or enzyme label [P.G. Colman, M. Tatkus, A. Rabizadeh, C. Cahill, and G.S. Eisenbarth: Assay for Islet Cell Antibodies with Rat Pancreas and Peroxidase Protein A. Diabetes Care 11:367-368 (1988)], and observing under a microscope. Another similar method involves a biotin-avidin sandwich and immunofluorescent detection [T. Kobayashi, T. Sugimoto, T. Itoh, K. Kosaka, T. Tanaka, S. Suwa, K. Sato and K. Tsuju: The Prevalence of Islet Cell Antibodies in Japanese Insulin-dependent and Non-insulin-dependent Diabetic Patients Studied by Indirect Immunofluorescence and by a New Method. Diabetes 35:335-340 (1986)]. These methods are time consuming, laborious, difficult to reproduce, and have limited sensitivity. The development of a more convenient immunoassay for ICA would permit widespread testing for epidemiology and correlation with IDDM, and ultimately prediction of the disease with a screening test.

A major limitation of current ICA tests is the limited knowledge and characterization of the islet cell antigens involved. The ICA's may be of low titer or affinity and approachable only with characterized antigens. ICA antigens that are detected by the immunofluorescence test are of special interest; these antigens may include:
(1) islet cell surface moieties [N.K. MacLaren, S.W. Hugng, and J. Fogh: Antibody to Cultured Human Insulinoma Cells in Insulin-dependent Diabetes. Lancet 1:997-1000 (1975), and A. Lernmark, Z.R. Freedman, C. Hofmann, A.H. Rubenstein, D.F. Steiner, R.L. Jackson, R.J. Winter and H.S. Traisman: Islet-cell-surface Antibodies in Juvenile Diabetes Mellitus. N. Engl. J. Med. 299:375-380 (1978)],
(2) insulin [J.P. Palmer, C.M. Asplin, P. Clemons, K. Lyen, O. Tetpati, P.K. Raghu and T.L. Paquette: Insulin Antibodies in Insulin-dependent Diabetics Before Insulin Treatment. Science 222:1337-1339 (1983), and S. Srikanta, A.T. Ricker, D.K. McCulloch, J.S. Soeldner, G.S. Eisenbarth and J.P. Palmer: Autoimmunity to Insulin, Beta Cell Dysfunction, and Development of Insulin-dependent Diabetes Mellitus. Diabetes 35:139-142 (1986)],
(3) a 64,000 dalton (64 kd) islet protein of unknown cellular localization [S. Baekkeskov, J.H. Nielsen, B. Marner, T. Bilde, J. Ludvigsson, and A. Lernmark: Autoantibodies in Newly Diagnosed Diabetic Children Immunoprecipitate Human Pancreatic Islet Cell Proteins. Nature 298:167-169 (1982)], and
(4) cytoplasmic antigens [G.F. Bottazzo, A. Florin-Christensen, and D. Doniach: Islet Cell Antibodies in Diabetes Mellitus With Autoimmune Polyendocrine Deficiencies. Lancet 2:1279-1283 (1974), A.C. MacCuish, J. Jordan, C.J. Campbell, L.J.P. Duncan, and W.J. Irvine: Antibodies to Islet-Cell in Insulin-Dependent Diabetics With Coexistent Autoimmune Disease. Lancet 2:1529-1533 (1974), R. Lendrum, G. Walker, and D.R. Gambli: Islet-Cell Antibodies in Juvenile Diabetes Mellitus of Recent Onset. Lancet 1:880-883 (1975), and W.J. Irvine, C.J. McCallum, R.S. Gray, G.J. Campbell, L.J.P. Duncan, J.W. Farquhar, H. Vaughan, and P.J. Morris: Pancreatic Islet Cell Antibodies in Diabetes Mellitus Correlated With The Duration and Type of Diabetes, Co-existent Autoimmune Disease, and HLA-type. Diabetes 26:138-147 (1977).
(5) glycoconjugates [R.C. Nayak, M.A.K. Omar, A. Rabizadeh, S. Srikanta, and G.S. Eisenbarth, "Cytoplasmic" Islet Cell Antibodies: Evidence That the Target Antigen is a Sialoglycoconjugate. Diabetes 34:617-619 (1985); P. Vardi, B.E. Dibella, T.J. Pasquarello, and S. Srikanta, Islet Cell Autoantibodies: Pathobiology and Clinical Applications. Diabetes Care 10:645-56 (1987); B.K. Gillard, J.W. Thomas, L.J. Nell and D.M. Marcus, Antibodies Against Ganglioside GT3 in the Sera of Patients with Type I Diabetes Mellitus. Journal of Immunology 142:3826-32 (1989)].

The 64 kd antigen has been characterized for size and isoelectric point by immunoprecipitation of radiolabeled islet proteins, but there is no sequence information. Two reports indicate a high prevalence of antibody that recognizes this protein in prediabetic sera as well as newly diagnosed diabetic sera [S. Baekkeskov, M. Landin, J.K. Kristensen, S. Srikanta, G. Jan Bruining, R. Mandrup-Poulsen, C. de Beaufort, J.S. Soeldner, G. Eisenbarth, F. Lindgren, G. Sundquist, and A. Lernmark: Antibodies to a 64,000 MW Human Islet Cell Antigen Precede the Clinical Onset of Insulin-dependent Diabetes. J. Clin. Invest. 79:926-934 (1987), and M.A. Atkinson, N.K. Maclaren, W.J. Riley, D.W. Sharp and L. Holmes: Mr 64,000 Autoantibodies (64KA) Predict Insulin Dependent Diabetes. American Diabetes Assoc. 48th Annual Meeting (1988) Abstract #391].

Some other molecular species have been characterized by Western blotting as being "common antigens" recognized by diabetic sera [D.G. Karounos, V.J. Virta, L.J. Nell, and J.W. Thomas: Analysis of Human and RINm5F Islet Cell Antigens. American Diabetes Assoc. Res. Symp. Woods Hole, MA. October 1987; Abstract #120]. These antigens have molecular weights of 150 kd, 84 kd, 60 kd, 49 kd, and 36 kd.

### SUMMARY OF THE INVENTION

The present invention provides a series of cloned nucleic acids that code for one or more proteins or protein fragments (pancreatic islet cell antigen) which bind selectively with pancreatic islet cell antibodies (ICA). Such cloned nucleic acids are characterized by the cDNA inserts in deposited recombinant bacteriophages ATCC 40550, 40551, 40552, 40553, 40554, 40703, 40704, 40705, and 40706.

The present invention, accordingly, also provides ICA proteins and peptide fragments thereof which are encoded by the cloned nucleic acids and are useful in the diagnosis of insulin-dependent (Type I) diabetes mellitus (IDDM). The ability of such proteins and peptides to bind to the antibody combining site on ICAs also confers utility in the binding or blocking of human immunoglobulin, T-cells or B-cells involved in IDDM, including circulating immunoglobulin, T-cells, and B-cells.

The ICA proteins and peptides of the present invention are obtained by such methods as full or partial expression, optionally with subsequent fragmentation, of the present cloned nucleic acids; and peptide or polypeptide synthesis based on the amino acid sequences determined from the present cloned cDNAs or from the full length ICA antigen genes that can be determined or isolated from islet cell nucleic acid libraries with the aid of the present complementary cloned cDNA sequences. Accordingly, such ICA proteins and peptides include the full length ICA proteins present in or on islet cells and which are expressed by the human gene whose mRNA is at least in part complementary with the complete sequence of the present cloned cDNAs. Also included in the ICA proteins and peptides of the present invention are the proteins expressed by recombinant cloning vehicles comprising the present cDNA inserts and fragments of such proteins obtained by partial expression or by subsequent fragmentation such as with restriction nucleases. The ICA proteins and peptides of the present invention also include peptides obtained by protein synthesis, such as those that are 3 amino acids in length or longer, which represent ICA epitopes or analogues or derivatives thereof.

The present invention offers a number of significant advantages. The molecular cloning of ICA antigens affords the preparation of large and reproducible amounts of material for use in research, diagnosis, and treatment of IDDM, as well as the opportunity to study the biological mechanisms involved in islet cell destruction and the appearance of ICA. The availability of large quantities of pure antigen enables the development of highly sensitive and specific immunoassays which can be used to screen the general population for presymptomatic IDDM or a predisposition to develop IDDM.

### BRIEF DESCRIPTION OF THE DRAWINGS

Figs. 1-5, 14 and 15 are reactivity profiles of ATCC-deposited ICA clones prepared in accordance with the present invention with diabetic and normal sera under conditions described in the Examples.

Fig. 6 is a control profile using the cloning phage with no recombinant insert.

Figs. 7 and 16 are summaries of the sera profiles of ICA clones showing reactivity values assigned by visual interpretation of the profiles in Figs. 1-5 and 14-15, respectively.

Figs. 8-13, 17 and 18 are DNA and inferred protein sequences of particular ICA clones.

Fig. 19 shows the results of immuno-precipitation of one of the ICA clones with diabetic and normal sera.

### DESCRIPTION OF THE PREFERRED EMBODIMENTS

As used herein, the term "ICA antigens" shall be understood to refer to the proteins and peptides provided by the present invention even though it is recognized that in some cases peptide forms will not be "antigens" in the strict sense, i.e., they will be haptenic since they will require attachment to a conventional macromolecular carrier in order to stimulate the production of antibodies in a host animal.

Furthermore, the "cloned nucleic acids", "cloned ICA antigen sequences", "cDNA inserts", and like terms shall refer to the inserts in deposited recombinant phages ATCC 40550, 40551, 40552, 40553, 40554, 40703, 40704, 40705, and 40706, and also to other nucleic acid sequences of full length genes, or fragments of such sequences, comprising such deposited sequences. It will be recognized that one or more full length ICA antigens are characterized by homology with the above deposited cDNA inserts, however, it is possible that two or more of such cDNA inserts correspond to a single ICA antigen. For example, the insert in ATCC 40703 appears to encompass the inserts for both ATCC 40550 and ATCC 40554, and thus these three inserts may all correspond to different and/or overlapping portions of a single ICA antigen.

### Preparation of Cloned ICA Antigen Sequences

In general, the cloned ICA antigen sequences of the present invention are obtained by expressing human genes in a suitable recombinant cloning vehicle, e.g., bacteriophage, and probing the resulting gene library with IDDM serum to select antigens that are recognized by ICA antibodies. Recombinant antigens are then screened with a panel of diabetic and normal sera to determine the disease specificity of the identified clones.

The particular deposited clones were more particularly obtained by the following method (further details can be found in the Examples below). A human cDNA library was generated by extracting RNA from purified human islets. This RNA was fractionated by chromatography to separate poly-A mRNA from other RNA such as ribosomal RNA and fragments of degraded RNA. The separated mRNA was reverse transcribed with a commercially available cDNA kit (Bethesda Research Laboratories), ligated to Eco RI DNA linkers, and ligated into lambda gt-11 arms for in vitro packaging. The ligated lambda was packaged using a commercial kit (Stratagene) and then amplified on a bacterial lawn in a plate format.

The phage library was screened with antibodies from autoimmune patients with Type I diabetes. Agarose plates were spread with bacteria infected with the phage, and recombinant protein expression was induced chemically. The protein was deposited onto filters which were then probed with serum. Plaques that appeared to be positive were isolated from the agarose plates and purified through two rounds of isolation. Subsequent to cloning, the gt-11 phage was infected into a bacterial host for large scale expression. Specificity of the proteins expressed by the cloned cDNA was evaluated by Western blotting of bacterial extracts containing the cloned human protein. Preparative polyacrylamide gels were run and electroblotted onto membranes, the membranes were cut into strips, and then reacted with a series of normal and diabetic sera. The clones that generated proteins that reacted exclusively or predominantly with diabetic sera were selected.

### Recombinant Cloning Vehicles and Subcloning

As is conventionally known in the art, the cDNA transcripts of the present invention, such as library cDNA or cDNA inserts excised from a cloning vehicle, can be incorporated into a variety of recombinant cloning vehicles for amplification of sequences of interest and for expression of ICA antigens of interest. A recombinant cloning vehicle will be understood to be a biochemical molecule or structure, e.g., DNA, that allows insertion of polynucleotide sequences and replication of the inserted polynucleotide sequences when the vehicle is appropriately incorporated into a host cell. An expression vehicle additionally includes the property of expressing the protein encoded by the inserted polynucleotide. In an expression vector, the inserted ICA antigen sequence is operably linked to a suitable control sequence capable of effecting the expression of ICA antigen in a suitable host. The control sequence involved will vary according to the host and transformation method selected. These matters are within the ordinary skill of the art.

Suitable recombinant cloning vehicles include plasmids, viruses and bacteriophage, and integratable fragments of DNA (i.e., fragments integratable into the host genome by recombination). Expression vehicles are particularly preferred and are exemplified, without limitation, by bacterial pEMBL, pMMB, and pUK, yeast pAAH5, pYE4, and pAB112, mammalian pRSV, vaccinia derived vectors, papilloma derived vectors, retroviral vectors, and shuttle vectors such as pCDM8. For a review, see D.M. Glover, DNA Cloning: A Practical Approach (1985) IRL Press Ltd. Suitable host cells include procaryotes, yeast, and higher eucaryotic cells including mammalian cells.

Subcloning of cDNA inserts can involve excising the insert for ligation into a different cloning vehicle. The insert can be excised using the restriction enzyme corresponding to the linkers used in the original insertion or using restriction enzymes selected from a restriction map of the insert. The excised cDNA can be inserted into another suitable vector for sequencing, amplification, or expression as desired. Should the terminal restriction sites in the original cloning vehicle have been destroyed, other enzymes can be used to recover the insert and resulting flanking regions from the cloning vehicle deleted by conventional means.

### Full-Length Gene Cloning

Fragments of the cDNA inserts of the present invention can be used to isolate full-length cDNA or genomic DNA clones from appropriate libraries by standard methods. The target library is spread on plates, allowed to grow, transferred to filters, and reacted with DNA probes. Such DNA probes are generated from restriction fragments of the cDNA inserts by such methods as end labeling, nick translation, random primed transcription, or photochemical means. Oligonucleotides can be synthesized, labeled, and used as hybridization probes. RNA probes can also be generated from subcloned cDNA by transcription from appropriate templates.

Recombinant cloning vehicles, e.g., phage or plasmids, that appear to react with the partial cDNA clones are re-screened and then restriction mapped. Promising clones are then sequenced to confirm the hybridization of the original probes and to obtain extended sequence information on the larger fragment. If full-length clones are not obtained in this procedure, the complete sequence of the nucleic acid coding for the human gene can be pieced together from overlapping sequences of cloned fragments.

An alternative method for obtaining longer fragments, and possibly full-length clones, uses antibodies raised against ICA antigens expressed by partial clones. After identifying an antigen of interest, it can be used as an immunogen to raise monoclonal or polyclonal antibodies of high titer and affinity. Such antibodies will enable the detection of longer cDNA clones and cDNA clones present in lower amounts in the library.

### Antigen and Peptide Synthesis

ICA antigens, as defined herein, can be prepared in a number of different ways from the clones and sequence information provided by the present invention. One can simply express the proteins from ICA antigen clones obtained according to the present invention, particularly from the deposited clones. Such expressed proteins, or fragments or digestion products thereof, can be used as antigens for binding to islet cell antibodies. However, direct use of bacterial expression extracts may not be possible in some cases since human sera normally react nonspecifically with E. coli proteins. In such cases, the expressed ICA antigens can be isolated by conventional techniques such as electrophoretic separation followed by immobilization on membranes (Western blotting), or by column chromatography or affinity purification (e.g., anti-beta-galactosidase affinity resin chromatography or other conventional biochemical means, e.g., salt or temperature precipitation).

Alternatively, peptide fragments can be synthesized by well-known methods from the amino acid sequences deduced from experimentally determined DNA sequences of ICA antigen clones. Overlapping peptides can be synthesized and tested for reactivity with ICA sera. As reactive peptides are found, smaller peptides can be prepared in order to map the smallest reacting unit, i.e., the epitope.

### Methods

A principal use of the ICA antigens provided by the present invention is in the diagnosis and prediction of IDDM. In such a method, a blood sample, normally a serum sample, is reacted with a selected one or series of ICA antigens and immunoreactivity determined by any conventional technique. It is further contemplated that the immunoreactivity profile with different ICA antigens can provide diagnostically significant information concerning the nature of the disease, e.g., subtypes, the state of the disease, the proximity to onset of the disease, the efficacy of therapy, e.g., immune therapy, and the like.

A further use of the present ICA antigens is in the identification, marking, or specific destruction of autoreactive B-cells. If autoantibodies have a deleterious effect in IDDM, it is contemplated that anti-B-cell therapy can slow or stem the progress of the disease from prediabetes to clinical IDDM.

Another use of the present ICA antigens is in the identification of islet-reactive T-cell populations. ICA antigens can serve as stimulating antigens for T-cell culture, permitting significantly improved T-cell cloning, identification, and growth. It is contemplated that ICA T-cell detection can be significant in the diagnosis of the pre-diabetic state, and that monitoring the level of autoreactive T-cells can give an indication of the progress of the disease and the utility of immune modulating therapies. Further, the generation of ICA T-cell cultures can provide an in vitro model for designing diabetic therapies. Finally, it is contemplated that T-cell immunization can halt or retard autoimmunity by generating a humoral response against self-destructive elements.

The ability of ICA antigens to bind to human ICA immunoglobulin and T-cells can be used to block the binding of ICA to islet cells and islet cell components in vivo, and therefore are contemplated to provide a direct therapeutic effect.

The present invention will now be illustrated, but is not intended to be limited, by the following examples.

### EXAMPLES

### 1. cDNA Library

Islets of Langerhans were purified by Dr.'s Paul Lacy and David Scharp at Washington Univ., St. Louis, MO, USA, following a published procedure. [C. Ricordi, P.E. Lacy, E.H. Finke, B.J. Olack, and D.W. Scharp: An Automated Method for Isolation of Human Pancreatic Islets. Diabetes 37:413-420 (1988)]. Briefly, human pancreas was perfused with collagenase and then ground up. Ficoll gradient centrifugation was used to isolate the islets, which were then cultured for 1 week at room temperature. The islets were frozen and shipped.

Upon receipt, the islets were thawed, pooled, and washed. RNA was extracted using guanidinium thiocyanate and selectively precipitated with lithium chloride [G. Cathala, J.F. Savouret, B. Mendez, B.L. West, M. Karin, J.A. Martial, and J.D. Baxter: A Method for Isolation of Intact, Transtationally Active Ribonucleic Acid. DNA 2:329-335 (1983)]. About 770 »g of total RNA was obtained from each ml of centrifuged islets. Messenger RNA was purified using Pharmacia Oligo(dT)-cellulose Type 7 (Pharmacia Fine Chemicals, Piscataway, NJ, USA), following the procedure of Maniatis et al, [T. Maniatis, E.F. Fritsel, and J. Sambrook: Molecular Cloning, A Laboratory Manual (1982) Cold Spring Harbor Laboratory p. 197-198]. About 30 »g RNA was obtained after chromatography. In vitro translation using a BRL kit #8110 (Bethesda Research Laboratory, Gaithersburg, MD, USA), and ³⁵S-methionine showed a broad range of molecular weight proteins being produced.

A BRL #8267SA kit was used for cDNA synthesis. Ten (10) »g of poly-A⁺ RNA was used in the reaction. The ends were polished with T₄-DNA polymerase (Pharmacia), and the cDNA was methylated with Eco RI methylase (New England Biolabs, Beverly, MA, USA) and S-adenosyl methionine and ligated to Eco RI linkers. The cDNA was digested with Eco RI and run on a Biogel A15M column (BioRad Laboratories, Rockville Center, NY, USA) to separate the linkers and fragments.

The cDNA was ligated into lambda gt-11 arms and packaged with a Stratagene Gigapack Plus kit (Stratagene Cloning Systems, LaJolla, CA, USA). A library of approximately 8.5 x 10⁵ insert-containing clones was obtained (measured with 5-bromo-4-chloro-3-indolyl-β-D-galactopyranoside), and amplified on E. coli Y1090 (Stratagene).

### 2. Sera

Sera from newly diagnosed diabetics were obtained from Dr. William Riley at the University of Florida, Gainesville, FL, USA, and Dr. Alan Drash at the Children's Hospital of Pittsburgh, Pittsburgh, PA, USA. Normal (non-diabetic) sera were collected from individuals in the laboratory. Sera were multiply adsorbed with filters that were prepared either by (a) lysing lambda-infected E. coli with chloroform and soaking nitrocellulose filters in this lysate, or (b) preparing filters by overlaying filters soaked with isopropyl-β-thio-galactopyranoside (IPTG) on lambda-infected E. coli in a plate format, essentially in the same manner as screening the library. Sera were diluted 1/20-1/200 in blotto solution (5% Carnation non-fat dry milk, 10 mM Tris pH 8.0, 150 mM NaCl, 0.05% Tween-20, and 0.05% sodium azide) after crude fractionation as noted below.

### 3. Screening

The screening procedure is based on standard protocols (T.V. Huynh, R.A. Young and R.W. Davis: Constructing and Screening cDNA Libraries in Fgt 10 and Gft 11 in DNA Cloning. D.M. Glover ed. (1985) IRL Press p. 490-78). Filters were prepared by plating about 50,000 plaque forming units (pfu) of the library onto each of ten 150 mm agarose plates. After growth at 42°C for about 3 hours, filters (Nitrocellulose from Schleicher and Schuell, Keene, NH, USA) containing IPTG were laid onto the plates and growth was continued at 37°C for either 3-4 hours or overnight. Filters were blocked with a blotto solution and stored at 4°C.

Initially, all antibody reactions with filters were performed at room temperature for 3 hours. In later experiments, sera incubations were done overnight at 4°C in blotto solution without Tween-20, while secondary antibody reactions were done at room temperature for 1.5 hours. All incubations and washing were done on platform shakers with gentle rotation.

The library was screened with human antibody probes several times. In the first instance, antibody was purified from diabetic sera by HPLC. In the second and third, sera were precipitated with 50% ammonium sulfate and dialyzed. For the first and third screenings, a mixture of two sera were used for all rounds of purification. For the second screening, a mixture of 20 diabetic sera was used for the primary purifications. In a further screening, 22 sera were pooled, precipitated with ammonium sulfate and dialyzed, and the final working dilution of each serum was 1/500 in blotto without Tween 20.

After incubation in the diabetic sera, filters were washed 5-10 minutes each in Tris-buffered saline (TBS), TBS with 0.05% Tween-20, and then in TBS. Human antibody bound to the filters was detected by reaction with rabbit anti-human IgG conjugated to alkaline phosphatase (1/500 in blotto, Dakopatts antibody D-336 - DAKO Corp., Santa Barbara, CA, USA). Filters were washed in TBS/Tween-20, TBS, TBS, and then detection buffer (0.1 M Tris-HCl, pH 9.5, 0.1 M NaCl, 0.05 M MgCl₂, recommended by BRL for use in their DNA detection kit No. 8239SA). Chromogenic substrates (nitro-blue tetrazolium and 5-bromo-4-chloro-3-indolyl phosphate) were added and the reaction was protected from light. After color development, the filters were washed in water, then in 10 mM Tris (pH 8.0), 1 mM EDTA (TE) and dried. Best observation of the plaques could be made when the filters were still matte wet.

Positive plaques were located on the original plates by alignment with the filters. For primary screens, a plug containing a positive plaque was removed using the butt end of a sterile Pasteur pipet. For subsequent screenings where individual plaques could be distinguished, the tip of the pipet was used. Plaques were eluted into plaque storage buffer (Maniatis et al., supra) and eluted for at least several hours.

The above screening methods produced the specific deposited ICA clones described herein with the exception of ICA-12.3 which was isolated as follows.

Approximately 10⁶ plaque forming units of the phage library were screened by DNA hybridization for the presence of sequences homologous with the ICA-12 cDNA. The phage plaques distributed over 20 agar plates were replicated onto nylon filters, and the phage DNA was denatured and immobilized for hybridization, by the conventional procedure of Benton and Davis (1977) Science 196:180. The hybridization probe was an agarose gel-purified sample of the cloned ICA-12 cDNA separated from its plasmid vector by Eco RI digestion. The cDNA segment was tagged with ³²P by the random primer labeling method (Feinberg and Vogelstein (1984) Anal. Biochem. 137:266). Hybridization of the probe to nylon filters was done according to Berent et al. (1985) BioTech. 3:208. Phage plaques identified as containing DNA homologous with the ICA-12 probe were picked from the master plates, and the phage were replicated for a second round of hybridization screening. Individual plaques remaining positive for ICA-12 sequences were then characterized as to properties of cDNA inserts. The clone ICA-12.3 was found by DNA sequence analysis to contain the entire protein coding sequence of the mRNA partially represented in ICA-12.

### 4. Expression

The proteins expressed by individual clones were analyzed by expressing the clones in E. coli hosts. Initial expressions with clones identified as ICA-12 and ICA-13 were done with lysogens generated with the clones by standard means (Huynh, et al.). Subsequent expressions were done by infective expression into E. coli CAG-456 [M. Snyder, S. Elledge, D. Sweetser, R.A. Young, and R.W. Davis: Fgt-11: Gene Isolation with Antibody Probes and Other Applications, Meth. Enzymology 154:107-128 (1987)]. Cells were harvested and lysed by resuspension in Laemmli sample buffer [U.K. Laemmli, Nature 227:680 (1970)]. Better electrophoresis results were obtained when samples were sonicated to reduce the size of the DNA and reduce viscosity.

Protein gel electrophoresis and semi-dry electrotransfer onto either nitrocellulose (Schleicher and Shuell) or Immobilon (Millipore Company, Bedford, MA, USA) were performed. Gels were stained with Coomassie Blue and filters were detected by immunoreaction with the same sera used to screen the library as detailed above.

### 5. Clone Analysis

In order to assess the usefulness of the individual clones for diagnosis of IDDM, each clone was tested for reactivity with a panel of diabetic and normal sera. This was done by reacting each serum with a Western blot strip from each clone. Preparative gel electrophoresis was followed by semi-dry electrotransfer of the proteins to filters. Identical 3 mm strips were cut from the filters and exposed to the various sera. Localization of antigen bands was done by reference to analytical Western blots and strips reacted with anti-beta-galactosidase antibody (1/2000 monoclonal antibody from Promega, Madison, WI, USA). Antibody incubation and detection with secondary antibody were described above.

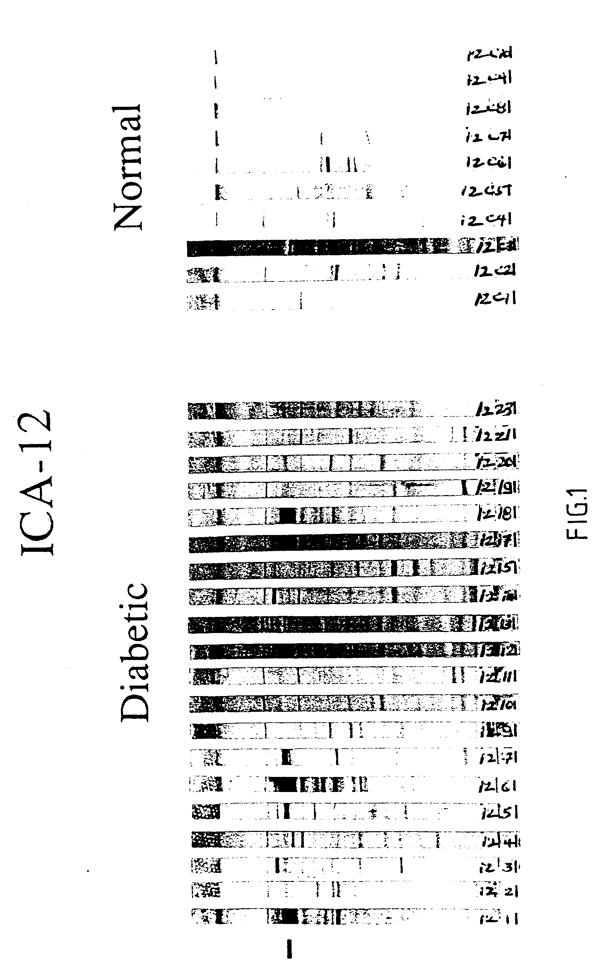

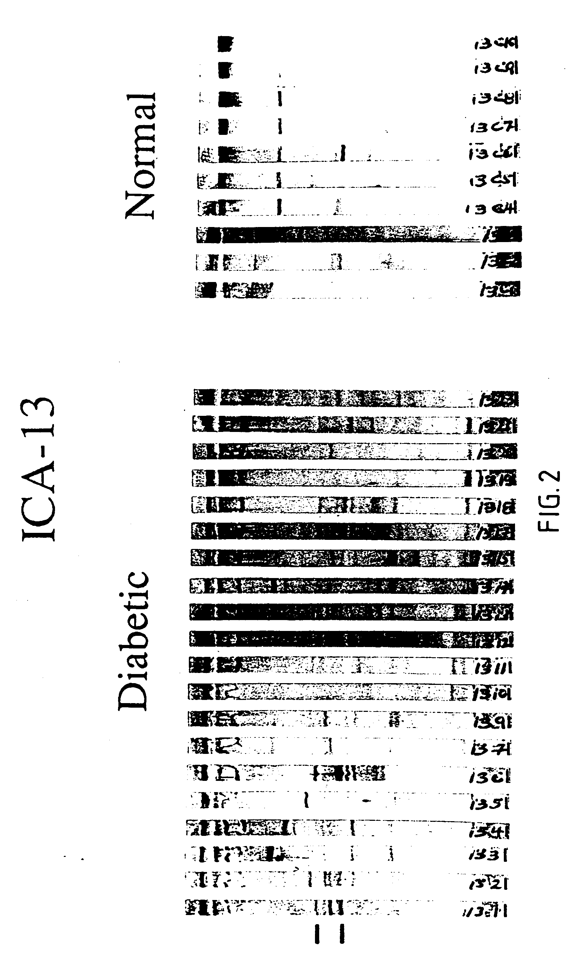

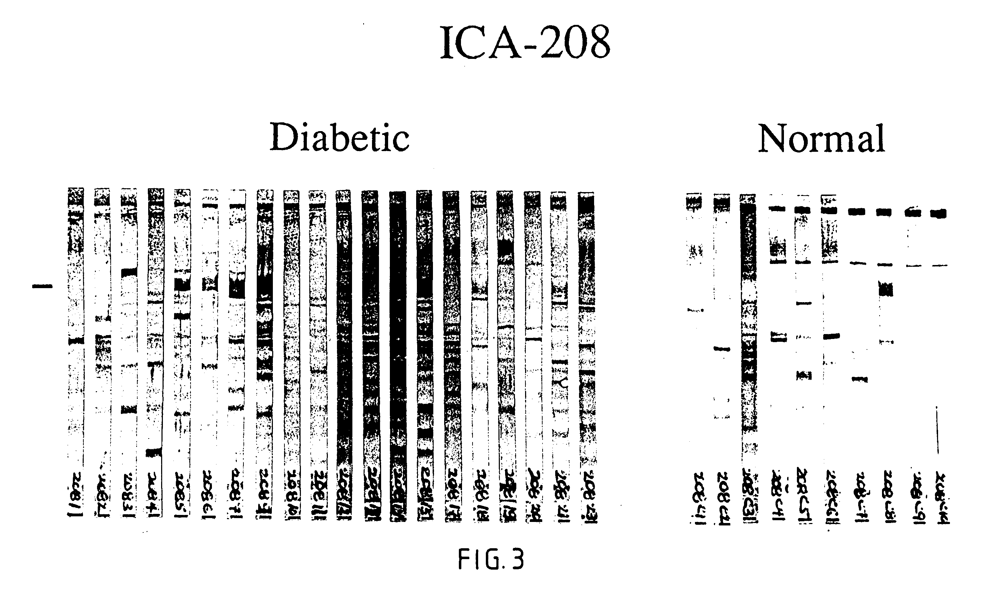

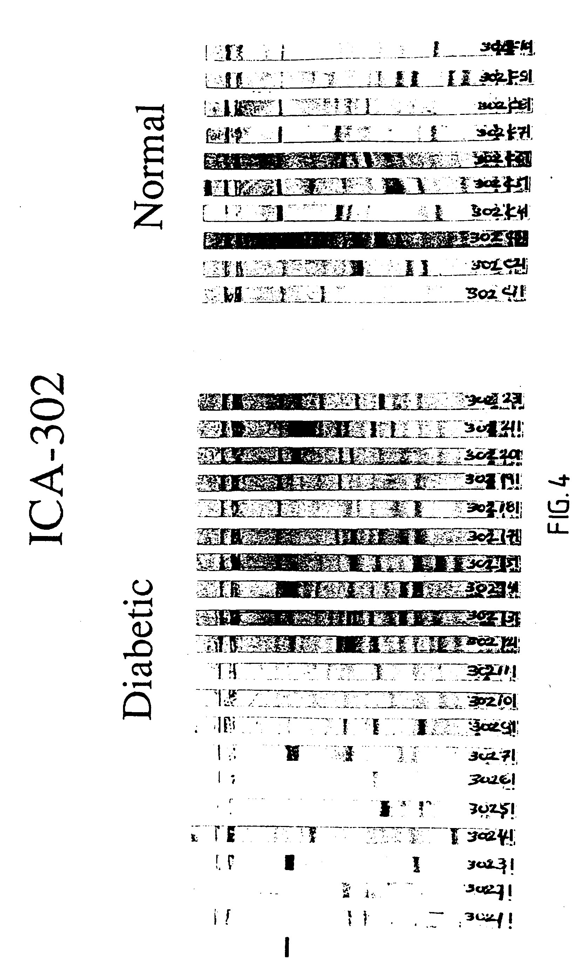

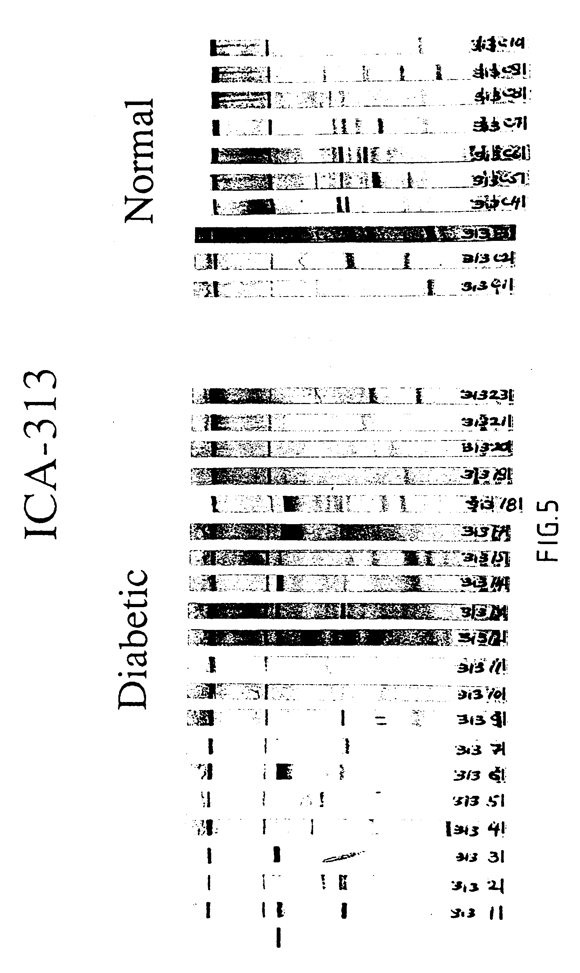

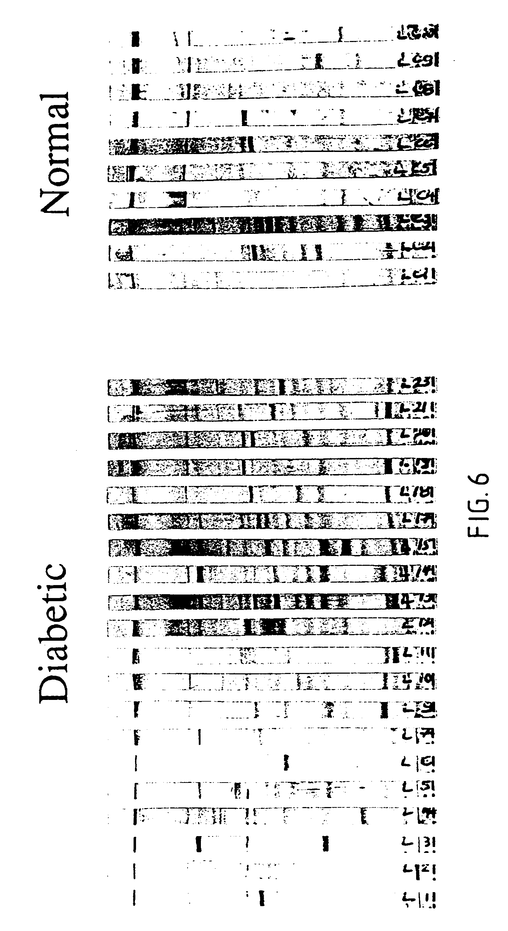

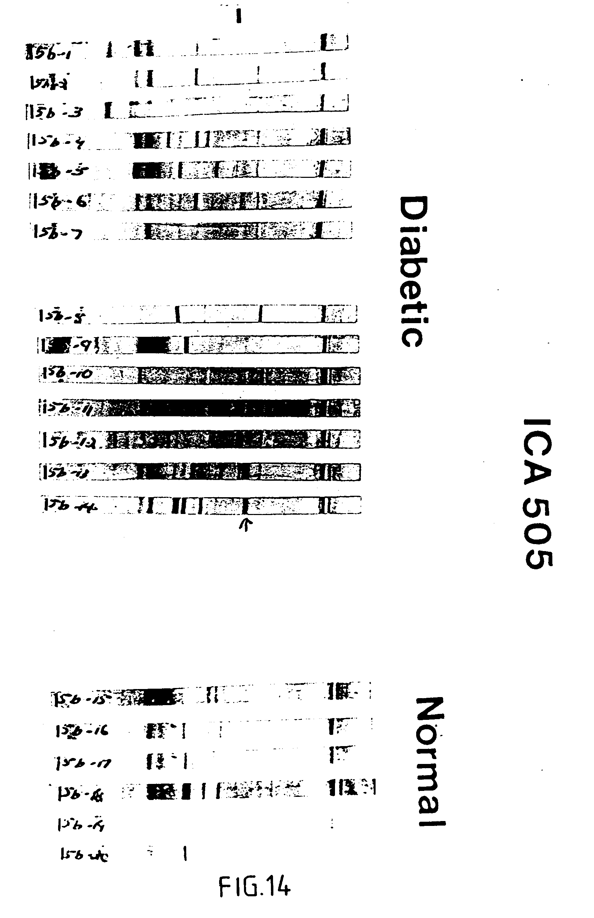

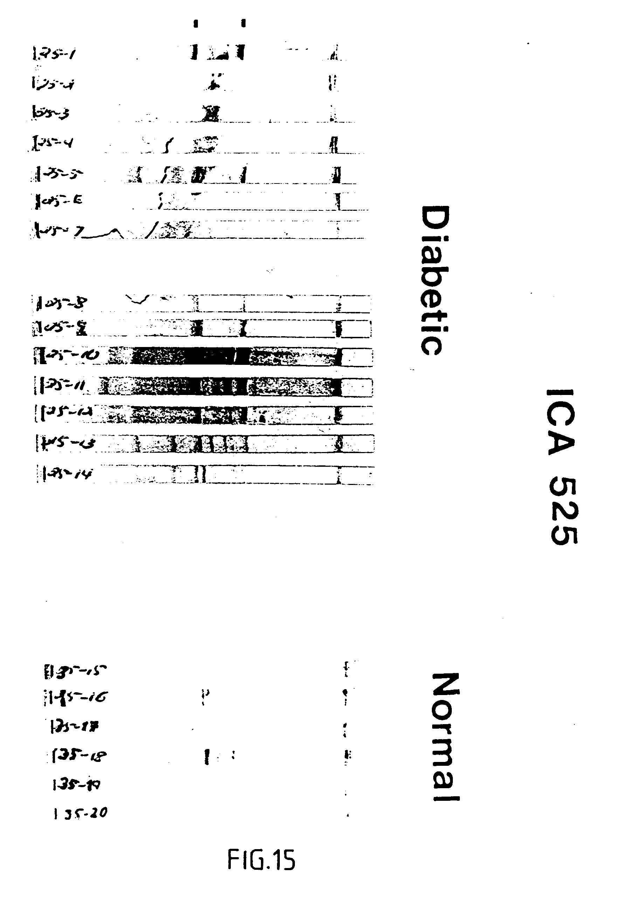

The reactivity profiles of the clones identified as ICA-12, ICA-13, ICA-208, ICA-302, ICA-313, ICA-505, and ICA-525 are shown in Figs. 1-5, 14 and 15 of the drawings. Identical filter strips were cut from preparative electrotransfer and reacted with diabetic and normal sera (for ICA-12, 13, 208, 302, and 313, the strips were reacted with 20 diabetic and 10 normal sera; while 14 diabetic and 6 normal sera were used for ICA-505 and 525). A control profile using the vector (lambda gt-11) having no DNA insert is shown in Fig. 6.

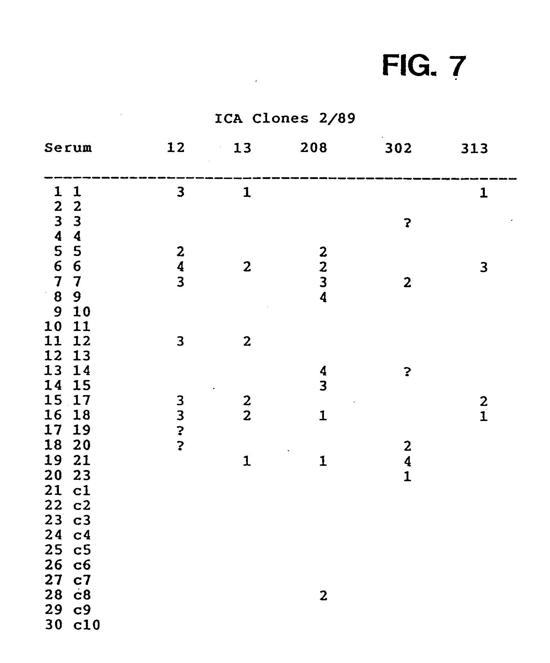

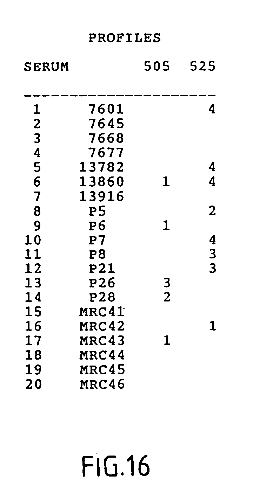

The filter strips were also rated according to intensity, 1 = weak reactivity, 2, 3 and 4 = very strong reactivity. Summaries of the reactivity ratings are given in Figs. 7 and 16 of the drawings. In Fig. 7, sera 21-30 bearing the prefix "c" are the normal control sera, while the diabetic sera are presented with their source identification number. In Fig. 16, sera 15-20 bearing the "MRC" prefix are normal control sera, while again the diabetic sera are presented with their source identification numbers. In both Figures, the numbers shown under the clone headings represent the strength of immunoreactivity assigned by visual interpretation.

Some clones identified in the first screening were found to be unreactive in the Western blot format. To test the serum reactivity of these clones, the lambda gt-11 phage were expressed in E. coli CAG456 as above and the antigen was extracted by treating the bacteria with 4 mg/ml lysozyme (Sigma, St. Louis, MO, USA) in 25 mM Tris, pH 8, 10 mM EDTA, 50 mM glucose and 2 mM phenyl-methyl sulfonyl chloride (PMSF) for 5 minutes at room temperature. Cells were pelleted at 4°C and resuspended in ice cold buffer (500 mM sodium chloride, 1% NP-40, 50 mM Tris, pH 8, 1 mg/ml aprotinin (Sigma), 2 mM PMSF, 2 »g/ml chymostatin (Sigma), 2 »g/ml Antipain (Sigma) and 2 »g/ml pepstatin. Extraction of antigen proceeded for 30 minutes on ice, during which time the solution was sonicated. Samples were spun in an Eppendorf microfuge for 5 minutes at 4°C and supernatants were used for immunoprecipitation.

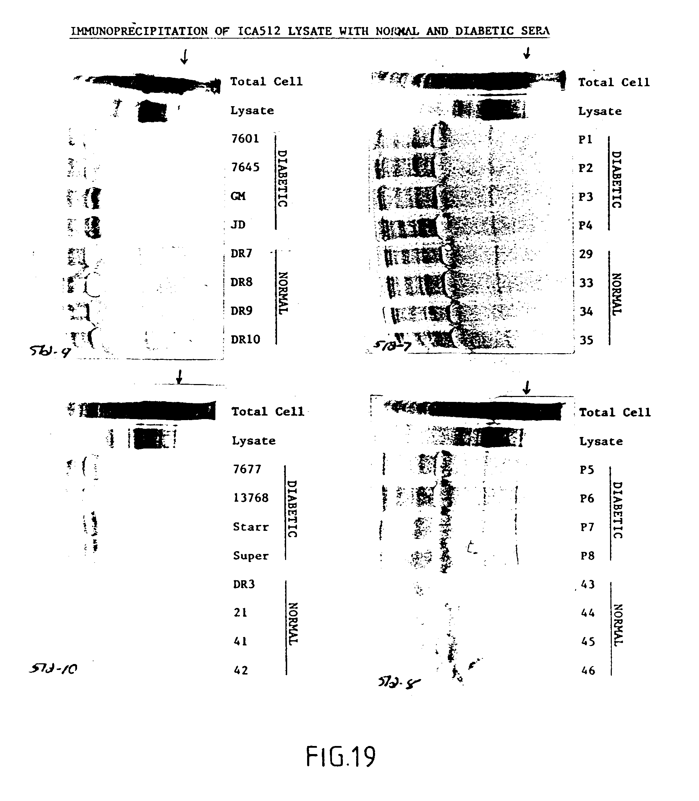

Immune reactions consisted of: 15 »l wash buffer (50 mM Tris, 150 mM sodium chloride, 1% NP-40, 5 mM EDTA, 2 mM PMSF, 2.5 »l human serum, and 10 »l extract. Reactions were left overnight. Antigen-antibody complexes were recovered with 20 »l of a 50% slurry of Protein-A Sepharose CL-4B (Pharmacia) for 1 hour on ice. The resin was washed six times with 500 »l of wash buffer and once with water. Sample buffer for PAGE was added and the samples were boiled for 5 minutes, centrifuged for 5 minutes, and run on 8% gels. Electroblotting was performed and the blots reacted with anti-beta-galactosidase antibody (1/1000 dilution of Sigma #G4644 in blotto solution) followed by anti-mouse Ig coupled to alkaline phosphatase (DAKO #D314) and development in dyes. The results are shown in Fig. 19 for an extract of ICA-512. Arrows indicate the position of the recombinant antigen.

DNA insert size for the various clones was determined by growing them either in a plate lysate or liquid lysate format (Maniatis, et al., supra). Lambda DNA was extracted, and cut with Eco RI, and analyzed for size by agarose gel electrophoresis.

The above identified clones which reacted predominantly with diabetic sera have been deposited with the American Type Culture Collection, Rockville, MD, USA. The deposit numbers and determined insert sizes are shown in Table 1 below.

**TABLE 1**

| Clone # | ATCC# | Insert size (kb) |
|---|---|---|
| ICA-12 | 40550 | 1.400 |
| ICA-13 | 40553 | 5.043 |
| ICA-208 | 40554 | 0.575 |
| ICA-302 | 40551 | 0.794 |
| ICA-313 | 40552 | 2.391 |
| ICA-12.3 | 40703 | 3.243 |
| ICA-525 | 40704 | 3.4 |
| ICA-505 | 40705 | 0.346 |
| ICA-512 | 40706 | 1.8 |

DNA inserts were transferred to a Stratagene Bluescript vector. Sequencing was done by standard techniques using the T7 Sequencing kit (Pharmacia) in conjunction with the Stratagene Exo III/Mung bean nuclease kit for generating overlapping nested deletion series of plasmids.

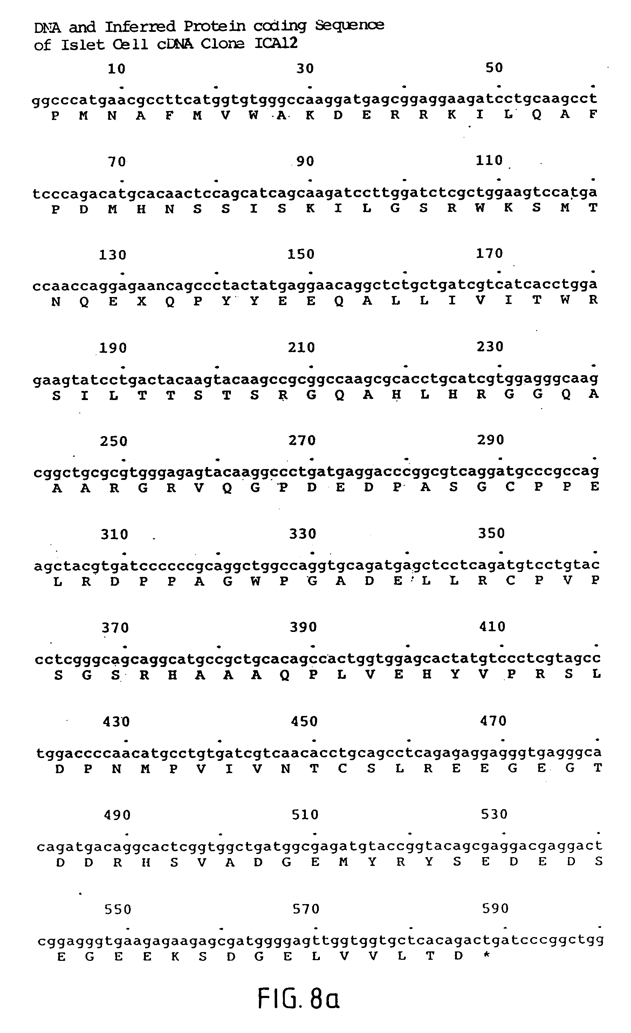

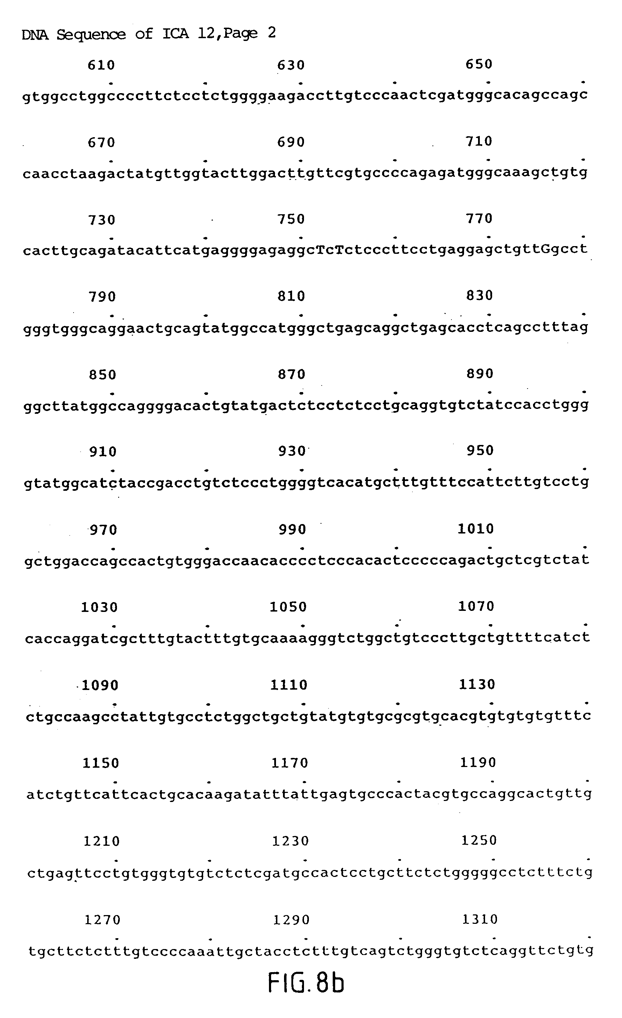

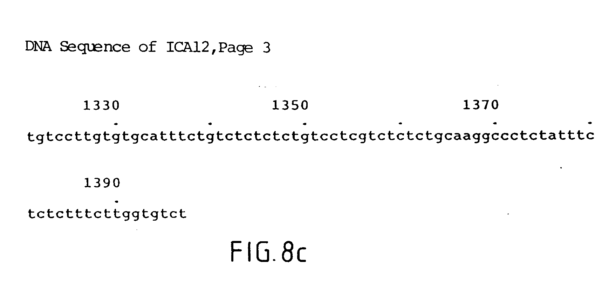

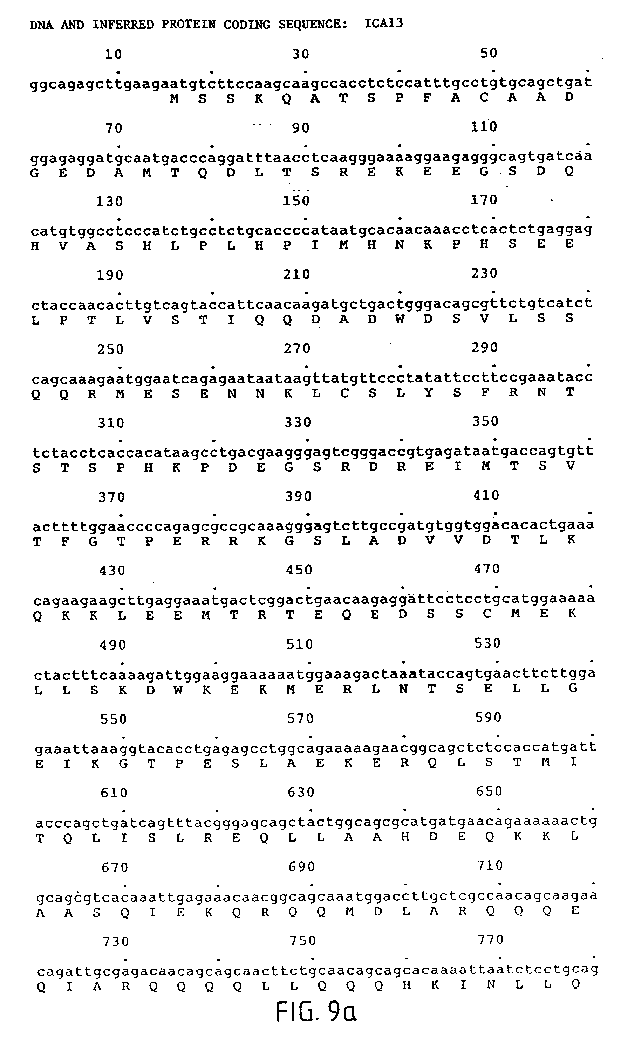

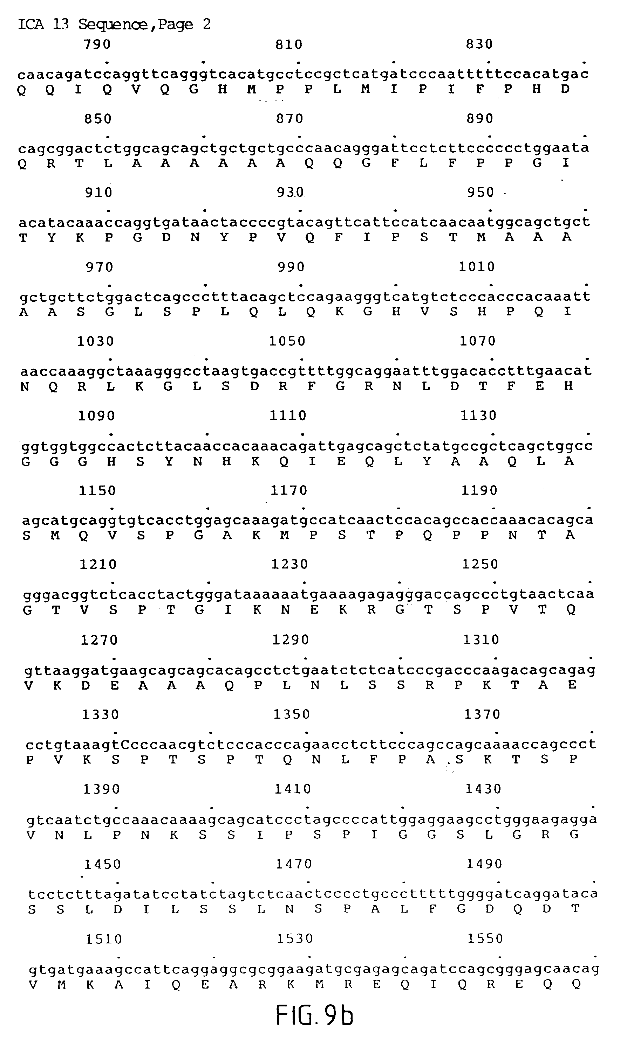

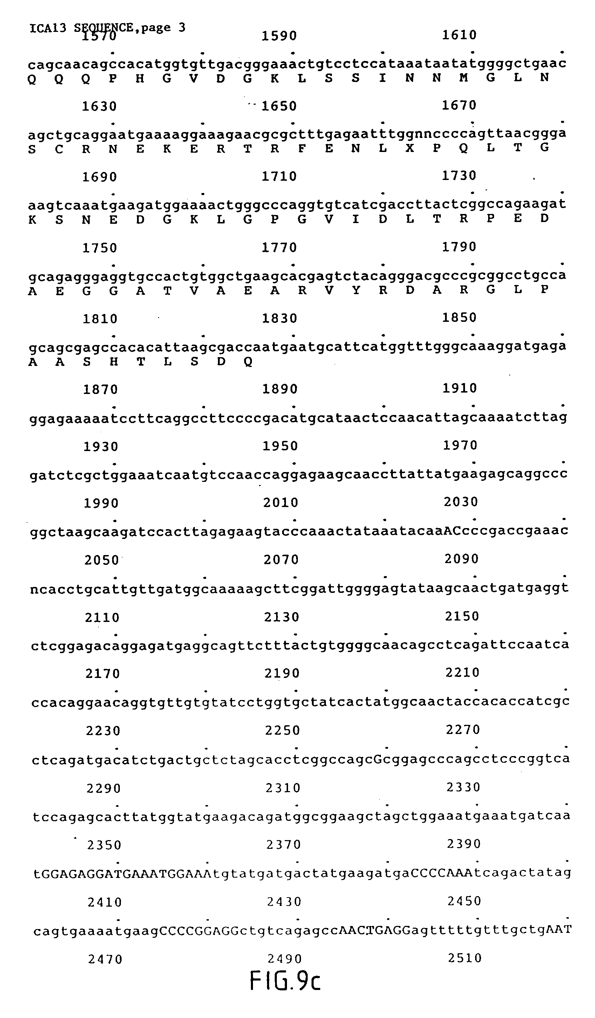

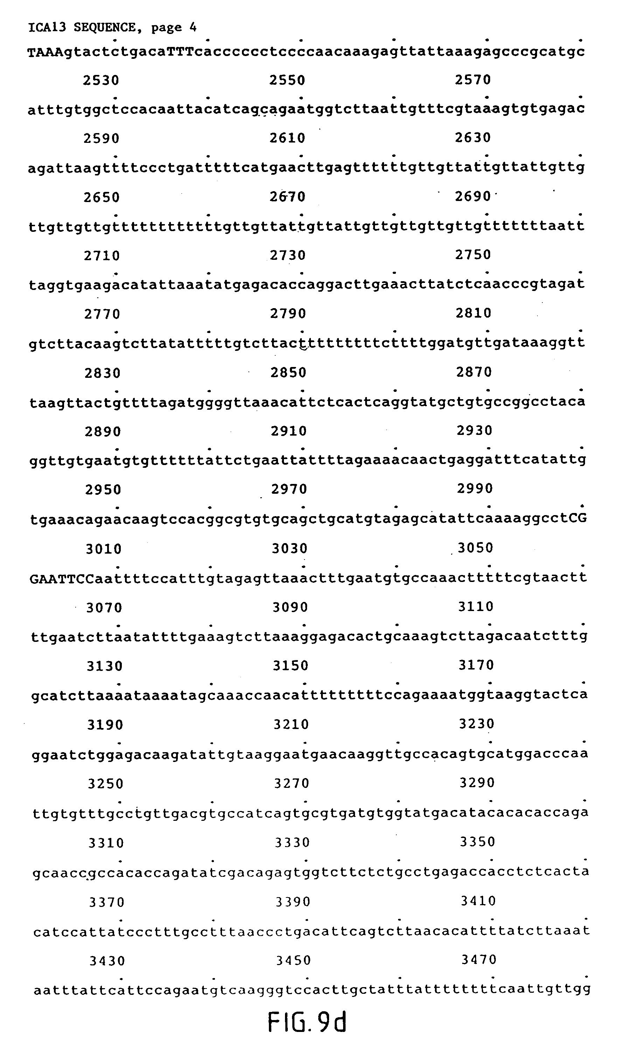

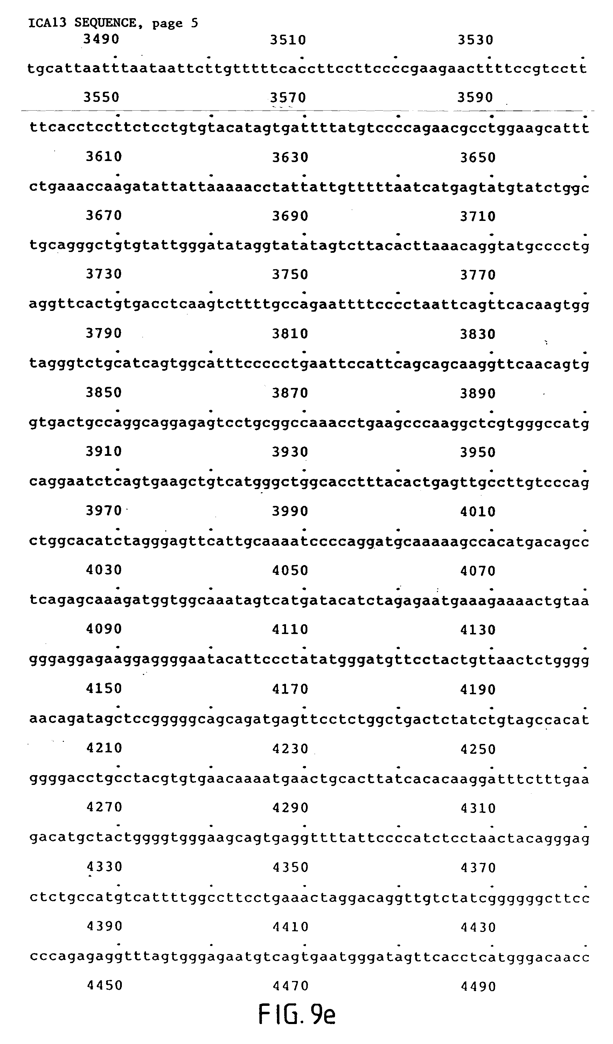

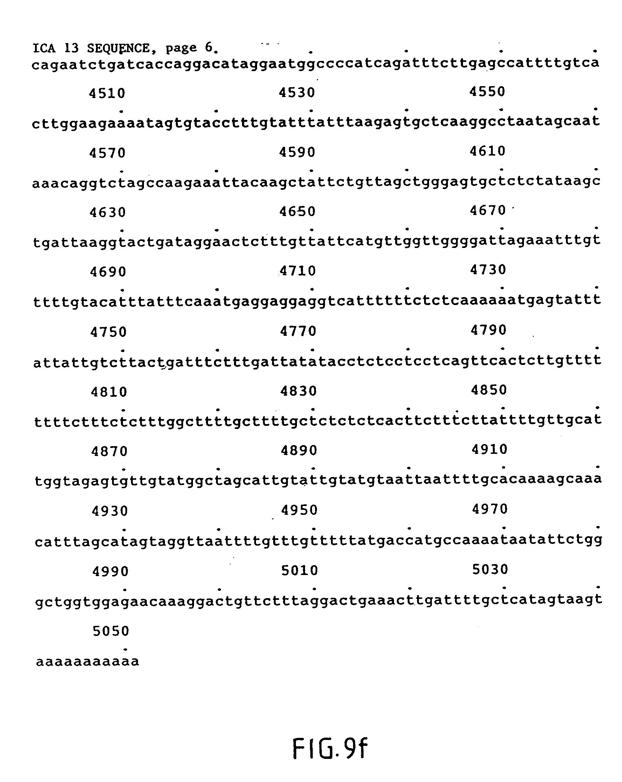

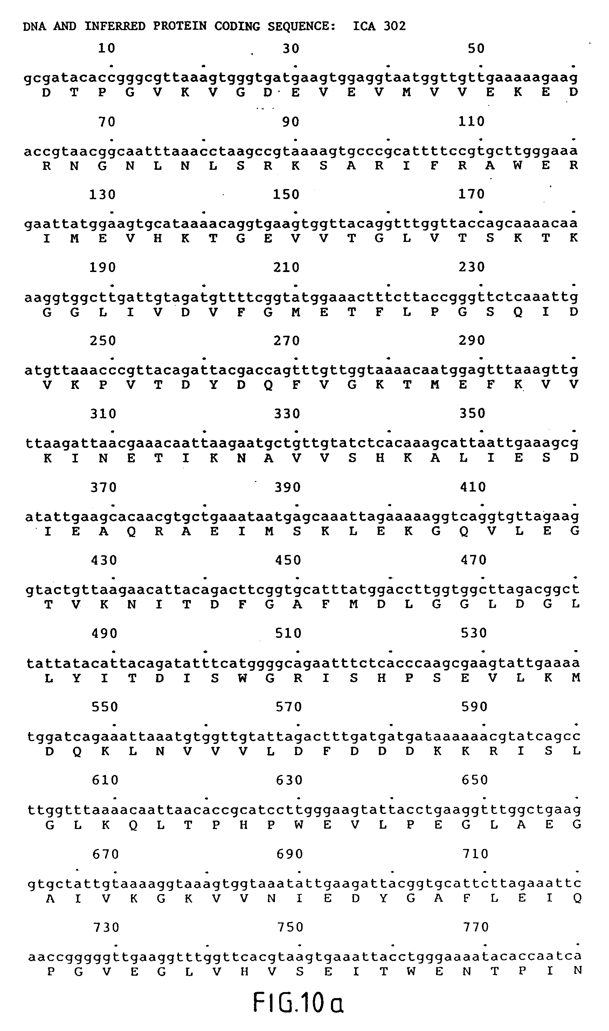

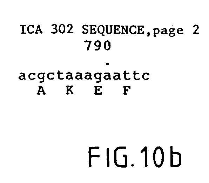

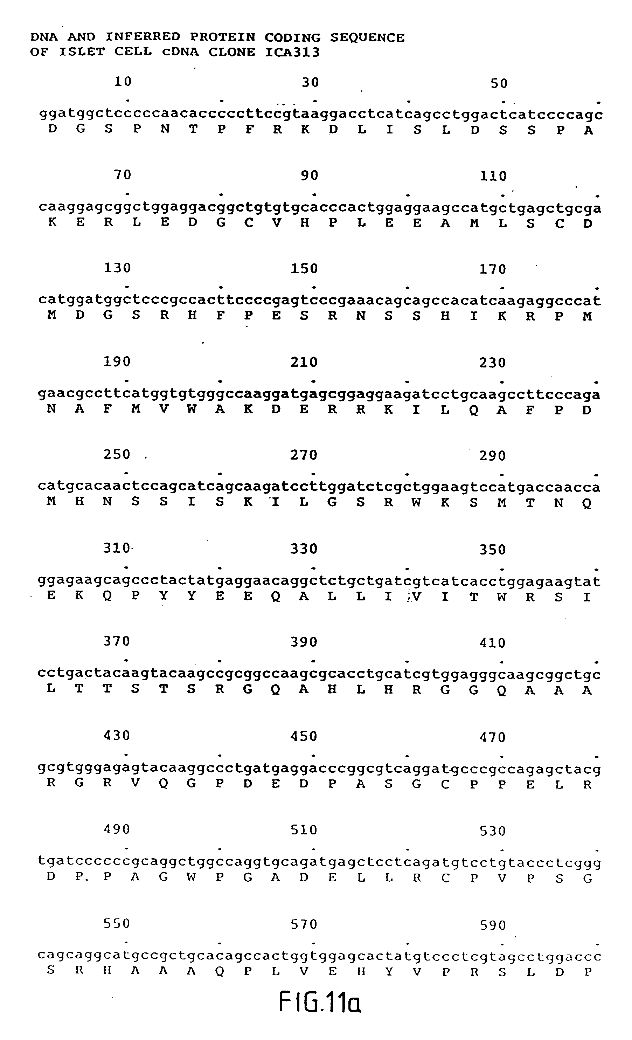

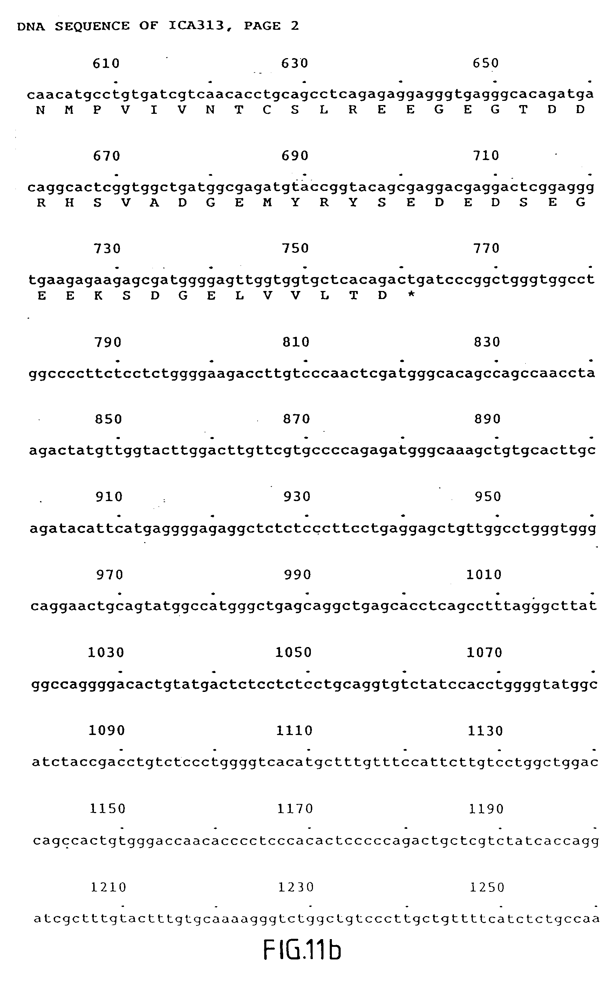

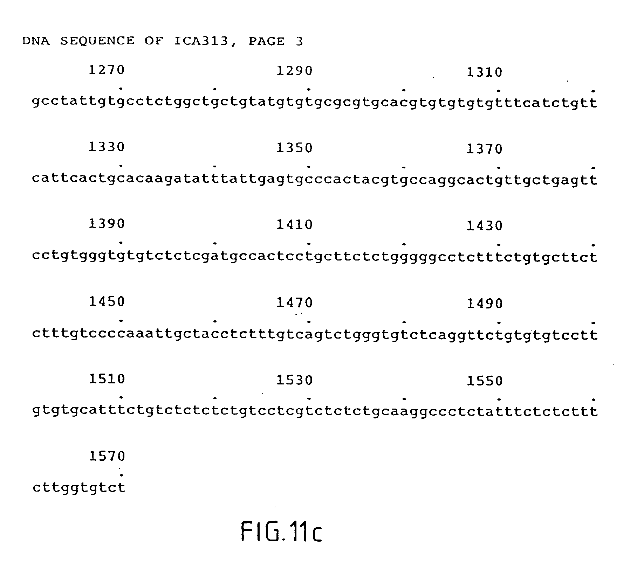

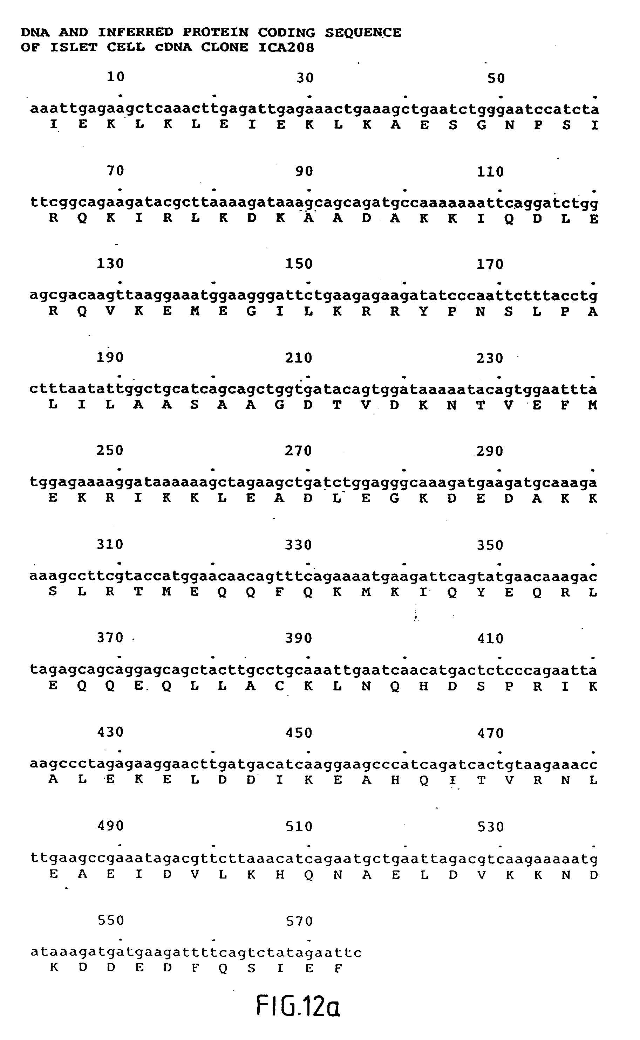

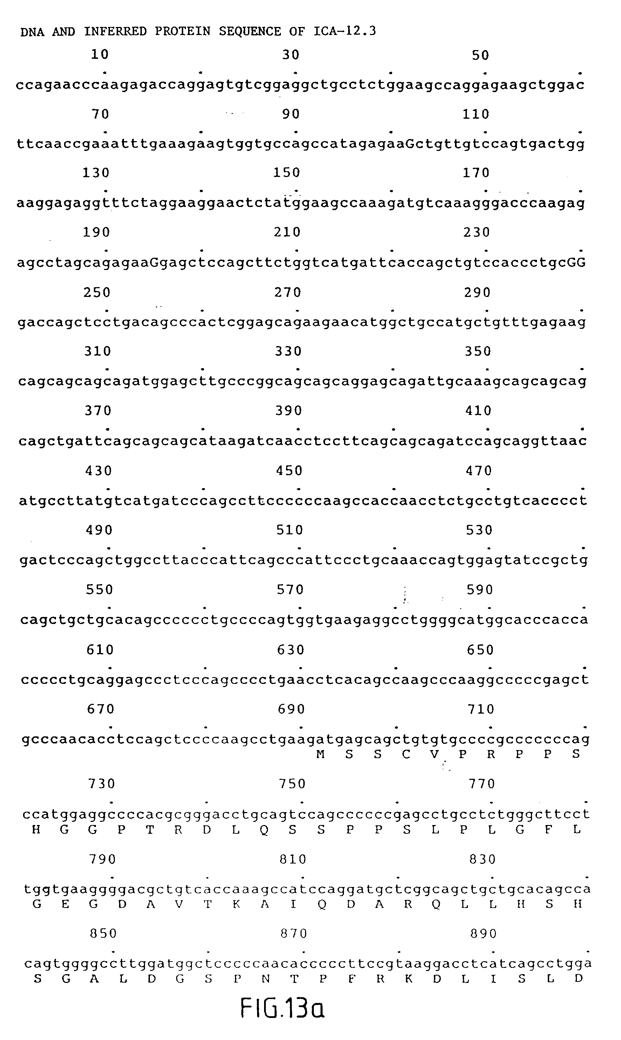

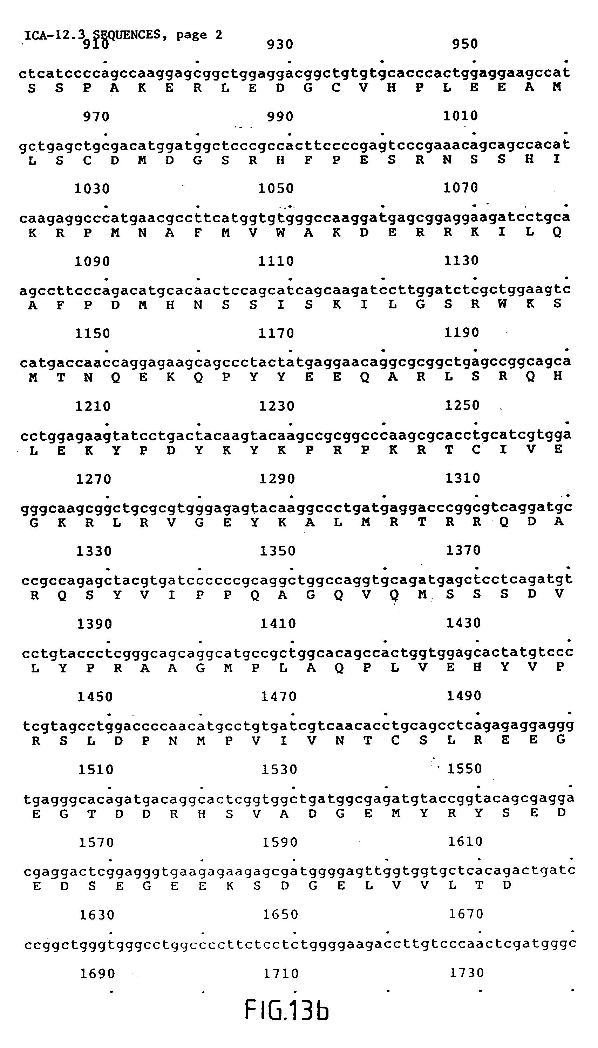

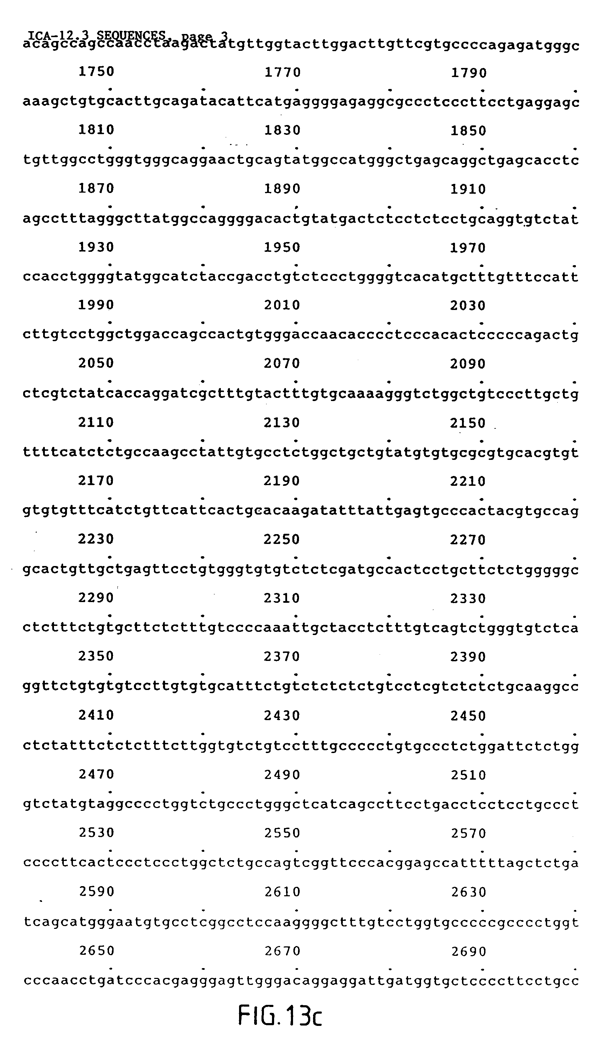

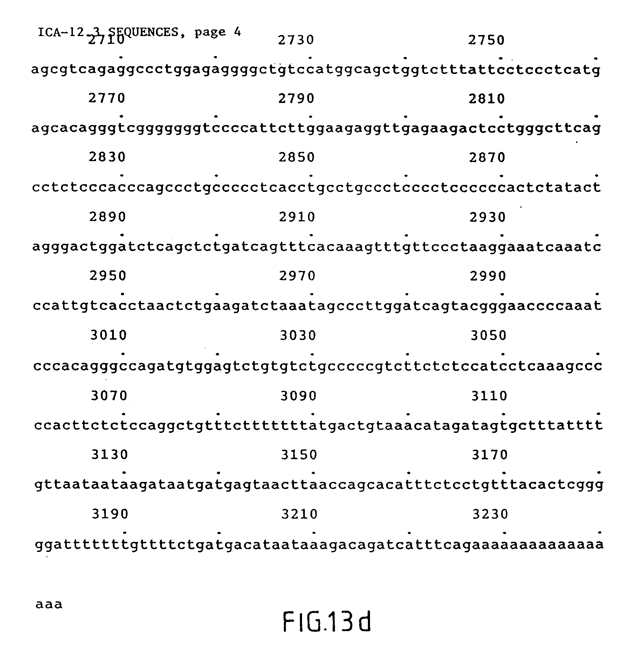

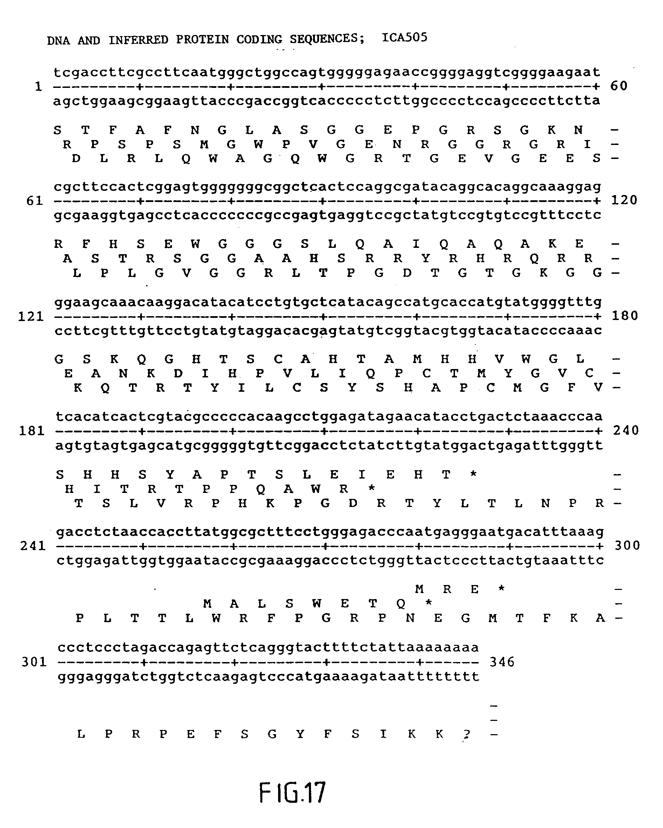

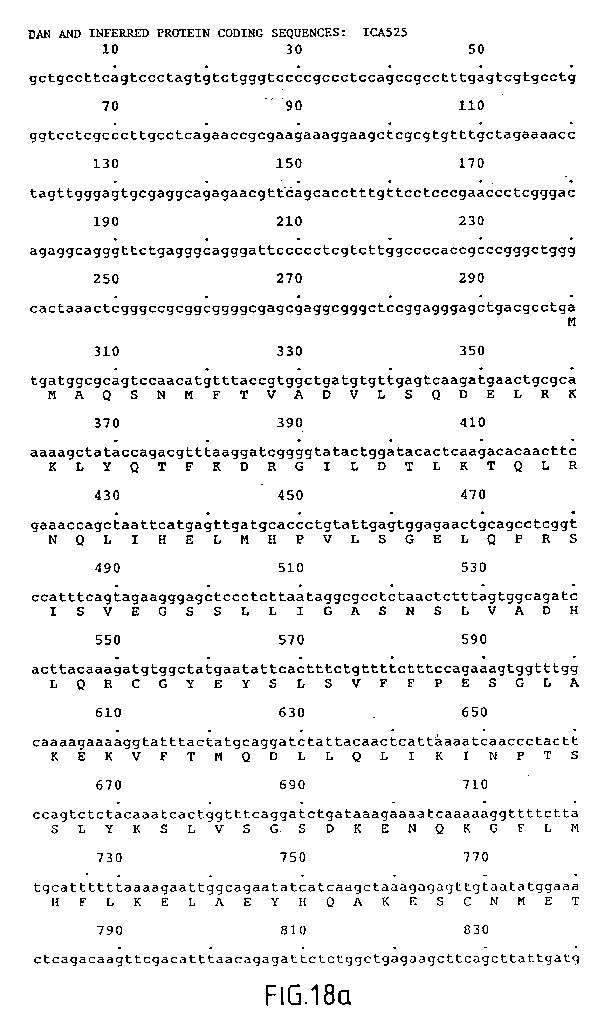

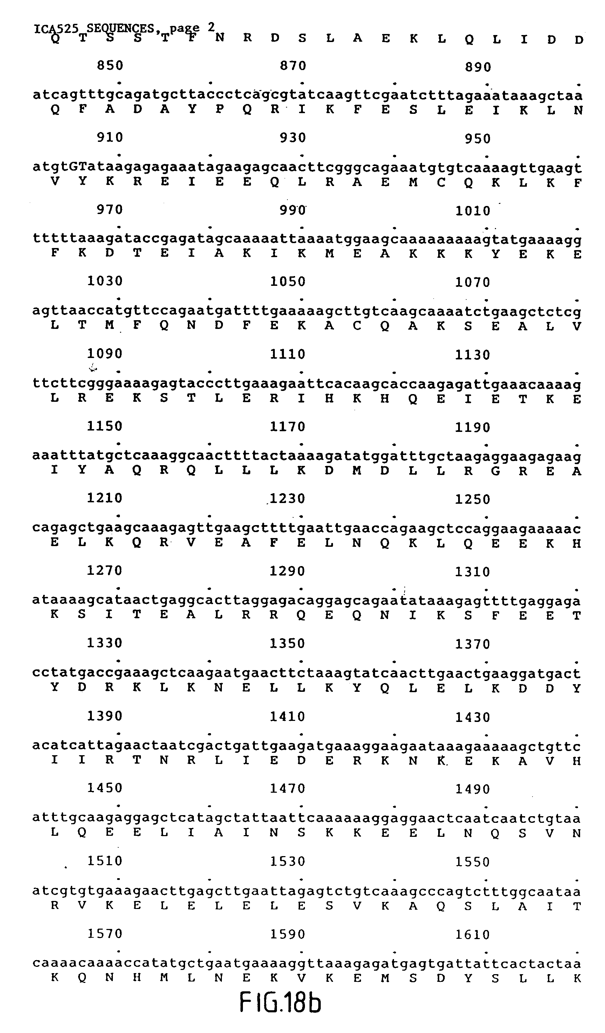

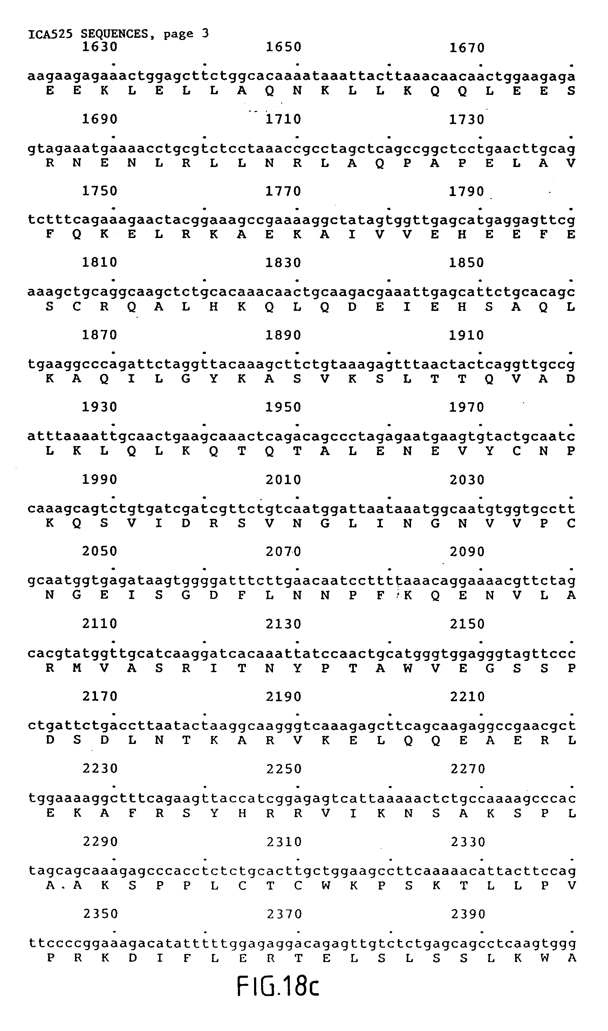

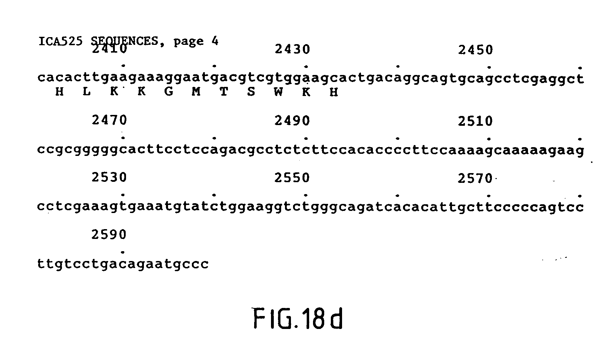

The available sequencing information on eight of the nine above-identified clones is given in Figs. 8-13, 17 and 18 of the drawings. The sequence is considered to be complete for ICA-12, 302, 313, 208, 505, 12.3, while only partial sequencing is available for ICA-13 and 525. The DNA sequences are those experimentally derived as described above. All three possible reading frames in both orientations were examined for protein coding capability, i.e., long open read frames. The most likely protein sequence for each clone is presented in capital letters below the DNA sequence (except for ICA-505 for which the available information does not permit assignment of the reading frame encoding the protein antigen).

The present invention has been particularly described and exemplified above. It is contemplated that many other variations and modifications of the invention can be made without departing from the spirit and scope hereof.

## Claims (Claims for the following Contracting State(s): AT, BE, CH, LI, DE, DK, FR, GB, IT, LU, NL, SE)

1. An isolated pancreatic islet cell antigen characterized in that it is related to insulin-dependent diabetis melitus and comprises the amino acid sequence encoded by the DNA insert of the recombinant cloning vehicle ATCC 40550, 40551, 40552, 40553, 40554, 40703, 40704, 40705 or 40706, or a fragment of such protein or peptide.

2. An isolated protein of claim 1 which is a full length protein expressed by the human gene whose mRNA is in part complementary with the complete sequence of the DNA insert of the recombinant cloning vehicle ATCC 40550, 40551, 40552, 40553, 40554, 40703, 40704, 40705, or 40706, or a protein or peptide fragment of such protein.

3. An isolated protein or peptide of claim 1 which has the amino acid sequence encoded by the DNA insert of the recombinant cloning vehicle ATCC 40550, 40551, 40552, 40553, 40554, 40703, 40704, 40705, or 40706, or which is a peptide portion of such sequence that is at least 3 amino acids long.

4. An isolated protein or peptide of any of claims 1 to 3 obtained either by expression in a recombinant DNA vector comprising an insert coding for such protein or peptide or by peptide synthesis.

5. A method of diagnosing insulin dependent diabetes mellitus in a patient, characterized in that a blood sample obtained from such patient is contacted with an antigen reagent comprising a protein or peptide of any of claims 1 to 4, and binding of antibody from the patient's blood sample to the antigen reagent is determined.

6. An isolated nucleic acid characterized in that it codes for a protein or peptide of any of claims 1 to 3, or an isolated nucleic acid hybridizable therewith.

7. A recombinant cloning vehicle characterized in that it comprises an insert that codes for a protein or peptide of any of claims 1 to 3.

8. A culturable cell characterized in that it has been transformed with a recombinant cloning vehicle of claim 7.

9. A method for detecting the presence of T-cells or B-cells that are involved in insulin dependent diabetes mellitus in a human blood sample, characterized in that the blood sample is contacted with the protein or peptide of any one of claims 1 to 4, and binding between such protein or peptide and T-cells or B-cells from the blood sample is determined.

10. A pharmaceutical preparation for blocking human immunoglobin, T-cells, or B-cells involved in insulin dependent diabetes mellitus comprising the protein or peptide of any one of claims 1 to 4.

## Claims (Claims for the following Contracting State(s): ES)

1. A method for detecting the presence of T-cells or B-cells that are involved in insulin dependent diabetes mellitus in a human blood sample, characterized in that the blood sample is contacted with an isolated pancreatic islet cell antigen which is related to insulin-dependent diabetis mellitus and comprises the amino acid sequence encoded by the DNA insert of the recombinant cloning vehicle ATCC 40550, 40551, 40552, 40553, 40554, 40703, 40704, 40705 or 40706, or a fragment of such protein or peptide, and binding between such protein or peptide and T-cells or B-cells from the blood sample is determined.

## Claims (Claims for the following Contracting State(s): GR)

1. An isolated pancreatic islet cell antigen which is related to insulin-dependent diabetis mellitus and comprises the amino acid sequence encoded by the DNA insert of the recombinant cloning vehicle ATCC 40550, 40551, 40552, 40553, 40554, 40703, 40704, 40705 or 40706, or a fragment of such protein or peptide.

2. An isolated protein of claim 1 which is a full length protein expressed by the human gene whose mRNA is in part complementary with the complete sequence of the DNA insert of the recombinant cloning vehicle ATCC 40550, 40551, 40552, 40553, 40554, 40703, 40704, 40705, or 40706, or a protein or peptide fragment of such protein.

3. An isolated protein or peptide of claim 1 which has the amino acid sequence encoded by the DNA insert of the recombinant cloning vehicle ATCC 40550, 40551, 40552, 40553, 40554, 40703, 40704, 40705, or 40706, or which is a peptide portion of such sequence that is at least 3 amino acids long.

4. An isolated protein or peptide of any of claims 1 to 3 obtained either by expression in a recombinant DNA vector comprising an insert coding for such protein or peptide or by peptide synthesis.

5. A method of diagnosing insulin dependent diabetes mellitus in a patient, characterized in that a blood sample obtained from such patient is contacted with an antigen reagent comprising a protein or peptide of any of claims 1 to 4, and binding of antibody from the patient's blood sample to the antigen reagent is determined.

6. An isolated nucleic acid characterized in that it codes for a protein or peptide of any of claims 1 to 3, or an isolated nucleic acid hybridizable therewith.

7. A recombinant cloning vehicle characterized in that it comprises an insert that codes for a protein or peptide of any of claims 1 to 3.

8. A culturable cell characterized in that it has been transformed with a recombinant cloning vehicle of claim 7.

9. A method for detecting the presence of T-cells or B-cells that are involved in insulin dependent diabetes mellitus in a human blood sample, characterized in that the blood sample is contacted with the protein or peptide of any one of claims 1 to 4, and binding between such protein or peptide and T-cells or B-cells from the blood sample is determined.

## Patentansprüche (Patentansprüche für folgende(n) Vertragsstaat(en): AT, BE, CH, LI, DE, DK, FR, GB, IT, LU, NL, SE)

1. Isoliertes pankreatisches Inselzellen-Antigen, dadurch gekennzeichnet, daß es mit Insulin-abhängiger Diabetes mellitus in Zusammenhang steht und die Aminosäure-Sequenz enthält, die durch das DNA-Insert des rekombinanten Klonierungsvehikels ATCC 40550, 40551, 40552, 40553, 40554, 40703, 40704, 40705 oder 40706 kodiert wird, oder Fragment eines solchen Proteins oder Peptids.

2. Isoliertes Protein gemäß Anspruch 1, das ein Protein in voller Länge ist, das durch das Humangen exprimiert wird, dessen mRNA teilweise komplementär mit der vollständigen Sequenz des DNA-Inserts des rekombinanten Klonierungsvehikels ATCC 40550, 40551, 40552, 40553, 40554, 40703, 40704, 40705 oder 40706 ist, oder Protein- oder Peptid-Fragment eines solchen Proteins.

3. Isoliertes Protein oder Peptid gemäß Anspruch 1, welche die Aminosäure-Sequenz aufweisen, die durch das DNA-Insert des rekombinanten Klonierungsvehikels ATCC 40550, 40551, 40552, 40553, 40554, 40703, 40704, 40705 oder 40706 kodiert wird, oder welche ein Peptidteil einer solchen Sequenz sind, der mindestens drei Aminosäuren lang ist.

4. Isoliertes Protein oder Peptid gemäß jedem Anspruch 1 bis 3, erhältlich entweder durch Expression in einen rekombinanten DNA-Vektor, der ein Insert enthält, das für ein solches Protein oder Peptid kodiert, oder erhältlich durch Peptid-Synthese.

5. Verfahren zur Diagnose Insulin-abhängiger Diabetes mellitus bei einem Patient, dadurch gekennzeichnet, daß eine von einem solchen Patient erhaltene Blutprobe mit einem Antigen-Reagens zusammengebracht wird, das ein Protein oder Peptid eines jeden Anspruchs 1 bis 4 enthält, und die Bindung eines Antikörpers aus der Blutprobe des Patienten an das Antigen-Reagens ermittelt wird.

6. Isolierte Nukleinsäure, dadurch gekennzeichnet, daß sie für ein Protein oder Peptid eines jeden Anspruchs 1 bis 3 kodiert, oder eine damit hybridisierbare isolierte Nukleinsäure.

7. Rekombinantes Klonierungsvehikel, dadurch gekennzeichnet, daß es ein Insert aufweist, das für ein Protein oder Peptid eines jeden Anspruchs 1 bis 3 kodiert.

8. Züchtbare Zelle, dadurch gekennzeichnet, daß sie mit einem rekombinanten Klonierungsvehikel gemäß Anspruch 7 transformiert worden ist.

9. Verfahren zum Nachweis des Vorliegens von T-Zellen oder B-Zellen, die in Insulin-abhängiger Diabetes mellitus involviert sind, in einer menschlichen Blutprobe, dadurch gekennzeichnet, daß die Blutprobe mit dem Protein oder Peptid eines jeden Anspruchs 1 bis 4 in Kontakt gebracht und die Bindung zwischen einem solchen Protein oder Peptid und T-Zellen oder B-Zellen aus der Blutprobe ermittelt werden.

10. Pharmazeutische Zubereitung zur Blockierung von Human-Immunoglobin, T-Zellen oder B-Zellen, welche in Insulin-abhängiger Diabetes mellitus involviert sind, enthaltend das Protein oder Peptid gemäß jedem Anspruch 1 bis 4.

## Patentansprüche (Patentansprüche für folgende(n) Vertragsstaat(en): ES)

1. Verfahren zum Nachweis des Vorliegens von T-Zellen oder B-Zellen, die in Insulin-abhängiger Diabetes mellitus involviert sind, in einer menschlichen Blutprobe,
dadurch **gekennzeichnet**, daß
die Blutprobe mit einem isolierten pankreatischen Inselzellen-Antigen, das mit Insulin-abhängiger Diabetes mellitus in Zusammenhang steht und die Aminosäure-Sequenz umfaßt, die durch das DNA-Insert des rekomoinanten Klonierungsvehikels ATCC 40550, 40551, 40552, 40553, 40554, 40703, 40705 oder 40706 kodiert wird, oder mit einem Fragment eines solchen Proteins oder Peptids in Kontakt gebracht und eine Bindung zwischen solchem Protein oder Peptid und T-Zellen oder B-Zellen der Blutprobe bestimmt werden.

## Patentansprüche (Patentansprüche für folgende(n) Vertragsstaat(en): GR)

1. Isoliertes pankreatisches Inselzellen-Antigen, das in Zusammenhang mit Insulin-abhängiger Diabetes mellitus steht und die Aminosäure-Sequenz umfaßt, die durch das DNA-Insert des rekombinanten Klonierungsvehikels ATCC 40550, 40551, 40552, 40553, 40554, 40703, 40705 oder 40706 kodiert wird, oder Fragment eines solchen Proteins oder Peptids.

2. Isoliertes Protein gemäß Anspruch 1,
das ein Protein in voller Länge ist, das durch das Humangen exprimiert wird, dessen mRNA teilweise komplementär mit der vollständigen Sequenz des DNA-Inserts des rekombinanten Klonierungsvehikels ATCC 40550, 40551, 40552, 40553, 40554, 40703, 40705 oder 40706 ist, oder Protein- oder Peptid-Fragment eines solchen Proteins.

3. Isoliertes Protein oder Peptid gemäß Anspruch 1,
welche die Aminosäure-Sequenz aufweisen, die durch das DNA-Insert des rekombinanten Klonierungsvehikels ATCC 40550, 40551, 40552, 40553, 40554, 40703, 40705 oder 40706 kodiert wird, oder welche durch einen Peptid-Teil einer solchen Sequenz dargestellt sind, die mindestens 3 Aminosäuren lang ist.

4. Isoliertes Protein oder Peptid gemäß jedem Anspruch 1 bis 3,
erhältlich entweder durch Expression in einem rekombinanten DNA-Vektor, der ein für ein solches Protein oder Peptid kodierendes Insert umfaßt, oder erhältlich durch Peptid-Synthese.

5. Verfahren zur Diagnose Insulin-abhängiger Diabetes mellitus bei einem Patienten,
dadurch **gekennzeichnet**, daß
eine von einem solchen Patienten erhaltene Blutprobe mit einem Antigen-Reagens in Kontakt gebracht wird, das ein Protein oder Peptid gemäß eines jeden der Ansprüche 1 bis 4 aufweist, und eine Bindung von Antikörper aus der Blutprobe des Patienten an das Antigen-Reagens bestimmt wird.

6. Isolierte Nukleinsäure,
dadurch **gekennzeichnet**, daß
sie für ein Protein oder Peptid gemäß eines jeden der Ansprüche 1 bis 3 kodiert, oder isolierte Nukleinsäure, die damit hybridisierbar ist.

7. Rekombinantes Klonierungsvehikel,
dadurch **gekennzeichnet**, daß
es ein Insert umfaßt, das für ein Protein oder Peptid gemäß eines jeden der Ansprüche 1 bis 3 kodiert.

8. Züchtbare Zelle,
dadurch **gekennzeichnet**, daß
sie mit einem rekombinanten Klonierungsvehikel gemäß Anspruch 7 transformiert worden ist.

9. Verfahren zum Nachweis des Vorliegens von T-Zellen oder B-Zellen, die in Insulin-abhängiger Diabetes mellitus involviert sind, in einer menschliche Blutprobe, dadurch **gekennzeichnet**, daß die Blutprobe mit dem Protein oder Peptid gemäß eines jeden der Ansprüche 1 bis 4 in Kontakt gebracht und eine Bindung zwischen solchem Protein oder Peptid und T-Zellen oder B-Zellen der Blutprobe bestimmt werden.

## Revendications (Revendications pour l'(les) Etat(s) contractant(s) suivant(s): AT, BE, CH, LI, DE, DK, FR, GB, IT, LU, NL, SE)

1. Antigène cellulaire isolé des îlots pancréatiques caractérisé en ce qu'il est apparenté au diabète sucré insulinodépendant et qu'il comprend la séquence d'aminoacides encodée par le produit d'insertion d'ADN du véhicule de clonage recombinant ATCC 40550, 40551, 40552, 40553, 40554, 40703, 40704, 40705 ou 40706, ou bien un fragment d'une protéine ou d'un peptide de ce type.

2. Protéine isolée selon la revendication 1, à savoir une protéine de longueur totale exprimée par le gène humain, dont l'ARNm est en partie complémentaire à la séquence complète du produit d'insertion d'ADN du véhicule de clonage recombinant ATCC 40550, 40551, 40552, 40553, 40554, 40703, 40704, 40705 ou 40706, ou bien un fragment protéinique ou peptidique d'une protéine de ce type.

3. Protéine ou peptide isolé selon la revendication 1, dont la séquence d'aminoacides est encodée par le produit d'insertion d'ADN du véhicule de clonage recombinant ATCC 40550, 40551, 40552, 40553, 40554, 40703, 40704, 40705 ou 40706, ou est une portion peptidique d'une séquence de ce type dont la longueur compte au moins 3 aminoacides.

4. Protéine ou peptide isolé selon l'une quelconque des revendications 1 à 3, que l'on obtient, soit par expression dans un vecteur d'ADN recombinant comprenant un produit d'insertion codant pour une protéine ou un peptide de ce type, soit par synthèse peptidique.

5. Procédé de diagnostic du diabète sucré insulinodépendant chez un patient, caractérisé en ce qu'un échantillon de sang obtenu auprès d'un tel patient est mis en contact avec un réactif antigénique comprenant une protéine ou un peptide selon l'une quelconque des revendications 1 à 4, et la liaison au réactif antigénique de l'anticorps de l'échantillon de sang provenant du patient.

6. Acide nucléique isolé caractérisé en ce qu'il code pour une protéine ou un peptide selon l'une quelconque des revendications 1 à 3, ou encore acide nucléique isolé apte à une hybridation avec elle ou avec lui.

7. Véhicule de clonage recombinant caractérisé en ce qu'il comprend un produit d'insertion qui code pour une protéine ou pour un peptide selon l'une quelconque des revendications 1 à 3.

8. Cellule cultivable caractérisée en ce qu'elle a été transformée avec un véhicule de clonage recombinant selon la revendication 7.

9. Procédé pour détecter la présence de cellules T ou de cellules B qui sont impliquées dans le diabète sucré insulinodépendant dans un échantillon de sang humain, caractérisé en ce que l'échantillon de sang est mis en contact avec la protéine ou le peptide selon l'une quelconque des revendications 1 à 4, et la liaison entre une protéine ou un peptide de ce type et les cellules T ou les cellules B provenant de l'échantillon de sang est déterminée.

10. Préparation pharmaceutique pour bloquer l'immunoglobuline humaine, les cellules T ou les cellules B impliquées dans le diabète sucré insulinodépendant, comprenant la protéine ou le peptide selon l'une quelconque des revendications 1 à 4.

## Revendications (Revendications pour l'(les) Etat(s) contractant(s) suivant(s): ES)

1. Procédé pour détecter la présence de cellules T ou de cellules B qui sont impliquées dans le diabète sucré insulinodépendant dans un échantillon de sang humain, caractérisé en ce que l'échantillon de sang est mis en contact avec un antigène cellulaire isolé des îlots pancréatiques qui est apparenté au diabète sucré insulinodépendant et qui comprend la séquence d'aminoacides encodée par le produit d'insertion d'ADN du véhicule de clonage recombinant ATCC 40550, 40551, 40552, 40553, 40554, 40703, 40704, 40705 ou 40706, ou bien un fragment d'une protéine ou d'un peptide de ce type et la liaison entre une protéine ou un peptide de ce type et les cellules T ou les cellules B provenant de l'échantillon de sang est déterminée.

## Revendications (Revendications pour l'(les) Etat(s) contractant(s) suivant(s): GR)

1. Antigène cellulaire isolé des îlots pancréatiques qui est apparenté au diabète sucré insulinodépendant et qui comprend la séquence d'aminoacides encodée par le produit d'insertion d'ADN du véhicule de clonage recombinant ATCC 40550, 40551, 40552, 40553, 40554, 40703, 40704, 40705 ou 40706, ou bien un fragment d'une protéine ou d'un peptide de ce type.

2. Protéine isolée selon la revendication 1, à savoir une protéine de longueur totale exprimée par le gène humain, dont l'ARNm est en partie complémentaire à la séquence complète du produit d'insertion d'ADN du véhicule de clonage recombinant ATCC 40550, 40551, 40552, 40553, 40554, 40703, 40704, 40705 ou 40706, ou bien un fragment protéinique ou peptidique d'une protéine de ce type.

3. Protéine ou peptide isolé selon la revendication 1, dont la séquence d'aminoacides est encodée par le produit d'insertion d'ADN du véhicule de clonage recombinant ATCC 40550, 40551, 40552, 40553, 40554, 40703, 40704, 40705 ou 40706, ou est une portion peptidique d'une séquence de ce type dont la longueur compte au moins 3 aminoacides.

4. Protéine ou peptide isolé selon l'une quelconque des revendications 1 à 3, que l'on obtient, soit par expression dans un vecteur d'ADN recombinant comprenant un produit d'insertion codant pour une protéine ou un peptide de ce type, soit par synthèse peptidique.

5. Procédé de diagnostic du diabète sucré insulinodépendant chez un patient, caractérisé en ce qu'un échantillon de sang obtenu auprès d'un tel patient est mis en contact avec un réactif antigénique comprenant une protéine ou un peptide selon l'une quelconque des revendications 1 à 4, et la liaison de l'anticorps de l'échantillon de sang provenant du patient au réactif antigénique est déterminée.

6. Acide nucléique isolé caractérisé en ce qu'il code pour une protéine ou un peptide selon l'une quelconque des revendications 1 à 3, ou encore acide nucléique isolé apte à une hybridation avec elle ou avec lui.

7. Véhicule de clonage recombinant caractérisé en ce qu'il comprend un produit d'insertion qui code pour une protéine ou pour un peptide selon l'une quelconque des revendications 1 à 3.

8. Cellule cultivable caractérisée en ce qu'elle a été transformée avec un véhicule de clonage recombinant selon la revendication 7.

9. Procédé pour détecter la présence de cellules T ou de cellules B qui sont impliquées dans le diabète sucré insulinodépendant dans un échantillon de sang humain, caractérisé en ce que l'échantillon de sang est mis en contact avec la protéine ou le peptide selon l'une quelconque des revendications 1 à 4, et la liaison entre une protéine ou un peptide de ce type et les cellules T ou les cellules B provenant de l'échantillon de sang est déterminée.
